(19) Europäisches Patentamt
European Patent Office
Office européen des brevets

(11) **EP 3 078 350 B1**

(12) **EUROPEAN PATENT SPECIFICATION**

(45) Date of publication and mention
of the grant of the patent:
**31.01.2018 Bulletin 2018/05**

(51) Int Cl.:
*A61F 2/24* *(2006.01)*          *A61F 2/90* *(2013.01)*
*A61F 2/01* *(2006.01)*

(21) Application number: **15162907.8**

(22) Date of filing: **09.04.2015**

(54) **3D FILTER FOR PREVENTION OF STROKE**

3D-FILTER ZUR VORBEUGUNG EINES SCHLAGANFALLS

FILTRE 3D POUR LA PRÉVENTION D'UN ACCIDENT VASCULAIRE CÉRÉBRAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**12.10.2016 Bulletin 2016/41**

(73) Proprietor: **Frid Mind Technologies
5032 Isnes (BE)**

(72) Inventor: **FRID, Noureddine
1650 Beersel (BE)**

(74) Representative: **Connor, Marco Tom et al
Pecher & Partners
Rue Louis de Geer, 6
1348 Louvain-la-Neuve (BE)**

(56) References cited:
**WO-A1-2013/082555      US-A1- 2003 100 940
US-A1- 2006 155 363      US-A1- 2009 125 098
US-A1- 2013 144 373**

## Description

## FIELD OF THE INVENTION

The present invention relates to implantable endoluminal prostheses and methods of using such devices in preventing clots migration to avoid ischemic strokes. More particularly, the present invention relates to devices further having a heart valve function that are designed to be placed in the ascending aorta including arch to prevent embolic material and blood clots from entering into the coronaries (the heart), supra aortic (the brain) as well as visceral branches (kidneys, lever etc.)

## BACKGROUND OF THE INVENTION

Strokes denote an abrupt impairment of brain function caused by pathologic changes occurring in upstream blood vessels. Sudden occlusion of an artery supplying blood to the brain causes ischemic stroke. Ischemia can also occur in any organs such as the kidneys, the liver and the heart.

About 20% of ischemic strokes are caused by cardio-embolism, and 44% are caused by atherosclerosis plaques. They are primarily caused by embolism of thrombotic material forming on the arterial or ventricular walls, or on the left heart valves. These thrombi come away and are swept along the arterial circulation. Cardio-embolisms are generally feared when cardiac arrhythmia or structural abnormalities are present. The most common cases of cardioembolic stroke are nonrheumatic atrial fibrillation (AF), prosthetic valves, rheumatic heart disease (RHD), ischemic cardiomyopathy, congestive heart failure, myocardial infarction, post-operatory state and protruding aortic arch atheroma.

Valvular heart disease is a major cause of morbidity and mortality in developing and industrialized countries. While rheumatic and infectious causes are more common in developing countries, degenerative valvular disease is the predominant etiology in the ageing population of the industrialized world. For patients with advanced, symptomatic disease, surgical open-heart valve replacement or repair remains the standard treatment with excellent short- and long-term outcomes.

There is, however, a significant percentage of typically older patients that are not considered as the best candidates for open surgery. For example, in Europe and the United States surveys, about 30% of patients with severe symptomatic aortic stenosis are not considered as surgical candidates owing to their advanced age and high rate of comorbidities. Because these patients have a poor outcome with medical management, less-invasive transcatheter approaches for valve repair/implantation, such as Transcatheter Aortic Valve Implantation (TAVI), appear promising for subgroups of these high-risk patients.

Stroke, however, remains a troublesome adverse event following TAVI. It is more frequent among patients who undergo TAVI than among patients submitted to surgical aortic valve replacement (SAVR) and is associated with reduced survival. Cerebrovascular accidents occur mostly during the procedure or shortly thereafter and are more frequent with repeated attempts to implant the prosthesis. TAVI causes a substantial amount of cerebral micro-emboli; importantly, the high number of micro-emboli may correlate with the severity of the post-procedural cerebral injury. The vast majority of embolic events and strokes are caused by embolization of atherosclerotic material and other debris tore away from the stenotic valve during various phases of TAVI.

Anticoagulants are a class of drugs commonly used to prevent the blood from forming critical clots that could result in a stroke. Anticoagulants are frequently used in patients who are already at high-risk for stroke such as patients who undergo TAVI or have atrial fibrillation (AF).

Warfarin belongs to a class of drugs called vitamin K antagonists, (VKAs) meaning that they interfere with the normal action of vitamin K, which is involved in the blood clotting process. Warfarin, the predominant anticoagulant in clinical use, reduces AF-related stroke by 64%, although this reduction is accompanied by an inherent risk of hemorrhagic complications, among which cerebral hemorrhage is especially serious. Thus up to 40% of patients with AF have the relative or absolute contraindications to anticoagulation therapy. The VKA has narrow therapeutic window and requires frequent laboratory monitoring of the international normalized ratio (INR) and subsequent dose adjustment to maintain patients within a goal INR.

The need for regular monitoring also results from the complicated pharmacokinetic profile of warfarin, the interactions with drugs, herbs, alcohol, and food, which can result in subtherapeutic (in inadequate stroke prophylaxis) or supratherapeutic (in bleeding events) drug levels. It was revealed that 44% of bleeding complications with warfarin were associated with supratherapeutic INR and that 48% of thromboembolic events occurred with subtherapeutic levels (Oake N, Fergusson DA, Forster AJ, van Walraven C. Frequency of adverse events in patients with poor anticoagulation: a meta-analysis. CMAJ. 2007;176(11):1589-94). Despite evidence-based recommendations for stroke prophylaxis with VKAs, they remain under prescribed in eligible patients with AF. Approximately 55% of patients with AF do not receive adequate stroke prophylaxis and, as result the incidence of stroke increased. Furthermore, patients who are actually treated with warfarin spend up to half of the treatment time outside the therapeutic range. This means that the full potential of warfarin to reduce stroke risk has never been fully realized nor achieved. However, Warfarin still has to be used because it has anti-dote in case of haemorrhagic event.

New oral anticoagulants (NOA) have been approved or are in development, and some are in the advanced stages of clinical research. NOAs act specifically by direct and irreversible inhibiting of the one coagulating factor. There are two classes of NOA; "direct thrombin (IIa) inhibitors" which inhibits enzyme thrombin, and "direct factor Xa inhibitors" which is central to propagation of coagulation. The NOAs have potential advantages over VKA, including a predictable anticoagulation effect that allows for fixed dosing, rapid onset and offset of action, and few drug and food interactions. In addition, they have a much wider therapeutic index compared with VKA, obviating the need for routine laboratory monitoring. However, if any bleeding occurred, the NOAs have no specific antidotes.

A few permanent filter devices have been reported for preventing embolic material from traveling the arteries directing to the brain, but these are not fully satisfied. For example, U.S. Patents Nos. 6673089 and 6740112 disclose a "self-expandable single-layer wire braided mesh" designed to be positioned at the bifurcation zone of the common carotid artery (CCA) to the external carotid artery (ECA). Theoretically, this braided mesh is deemed to deviate emboli to the ECA (bringing the blood in to the face) and avoid carrying it to the brain through the internal carotid artery (ICA). The rerouting efficacy of emboli into the external carotid artery (ECA) was assessed clinically by Sievert et al. in Cardiovas Intervent Radiol (2012) 35:406-412, "A novel carotid device for embolic diversion" in three patients during 6 to 14 months follow-ups and high risk of filter occlusion is observed in front of the ICA orifice. U.S. Patent Application Publication No. 2003/0100940 discloses a stent-like protector device for filtering emboli originating from upstream sources and preventing them from entering the aortic arch's side branches that carry blood to the brain. Said filtering device consists of single-layer mesh-like tube in the form of a braided structure made of 100-160 filaments having 50-100 $\mu$m of diameter, the mesh opening width being 400-1000 $\mu$m. U.S. Patent No. 5 061 275 discloses that a braided self-expanding single-layer stent has a limitation in the number of wires and diameter of wires in order to obtain a reasonable hoop force when it is deployed in a body lumen. Namely, the greater the diameter of prosthesis, the more critical this limitation becomes. For example, if the diameter of a prosthesis is 30 mm, the diameter of wires has to be between 220 and 300 $\mu$m and the number of the wires must reach 36 to 64 wires, otherwise the wall of the prosthesis cannot exerts a sufficient hoop force against the wall of the corresponding vessel. For obtaining a braided single-layer stent having a sufficiently large device diameter for to fit to an aorta region (e.g. 25 to 45 mm) and same time fine mesh openings, this stent should consist of either (i) a high number of wires having small diameter or (ii) a long length of wires forming more than 150 degree of angle between braided wires. Such parameters (i) and (ii), however, do not permit to braid stents offering the adequate hoop force required for implantation in the aortic arch as discussed in U.S. Patent No. 5 061 275. US-A-2013/144373 discloses an expandable braided mesh frame for the aortic arch.

The proximal end of the frame comprises a neck which has a circular cross-section being smaller than the remainder of the tubular body. A valve body having an impermeable material is placed within the neck portion.

## SUMMARY OF THE INVENTION

A first object of the invention is developing an easy method for replacement of a deficient heart valve.

Another object is to provide an implantable endoluminal prosthesis which limits and/or prevents the spreading of blood clots and embolic materials formed in the left ventricle, in the ascending aorta or on the aortic valve, throughout body vessels, especially in the direction of the heart via coronaries and via supra aortic branches.

Another object of the invention is to provide an implantable endoluminal prosthesis with a heart-valve function which ensures a firm support for the heart valve and stabilizes the valve post-implantation.

It is another object of the invention to provide an implantable endoluminal prosthesis suitable to be deployed within a curved vessel such as the aortic arch in front of branches supplying blood to all bridged vessels as those oxygenating the brain, and further suitable to deflect effectively embolic material that would have flown into the aortic arch branches, into the descending aorta, thereby preventing extracranial embolus from occluding small intercranical arteries in the brain.

It is another object of the invention to provide a method for treating patients known to suffer from embolic diseases, by selectively occluding the passage of embolic material within the aortic arch and deviating it from the aortic arch branches.

It is another object of the present invention to provide an implantable filtering medical device able to provide substantially same maximal mesh opening size when deployed in a curved lumen as the one in its expanded state, thus suitable to be positioned in an aortic arch while keeping an adequate surface coverage ratio and mesh opening size at the outer side of the curve so as to obtain sufficient emboli rerouting efficacy.

It is still another object of the present invention to provide an implantable medical device and a method for improving the perfusion of organs by the lamination through the device, such as the brain, the kidneys, the liver and the heart, wherein the inlet of branch leading to said organ is covered with the implantable medical devices.

The subject of the present invention is defined in the appended independent claim. Preferred embodiment are defined in the dependent claims.

A subject of the present invention is an implantable endoluminal prosthesis suitable for deployment from the aortic annulus to the aorta. The prosthesis comprises a self-expandable braided framework able to expand from a radially compressed state in a delivery configuration to a radially expanded state. The self-expandable braided framework is formed of braided wires having a given diameter ($\emptyset_{25}$), and has a proximal end configured to extend toward the heart and a distal end configured to extent toward away from the heart. The self-expandable braided framework extends along an axis. The self-expandable braided framework comprises a main tubular body at the distal end of the self-expandable braided framework, a neck at the proximal end of the self-expandable braided framework, and a transition portion extending between the proximal end of the main tubular body and the distal end of the neck. The main tubular body and the neck comprises respectively a lumen in a cylindrical form with a circular cross-section and a constant diameter. The diameter of neck is smaller than the one of main tubular body. The main tubular body, the neck and the transition portion consist of an integrated structure being devoid of any impermeable cover layer, and forms a wall having a thickness ($T_{20}$). The prosthesis further comprises a radially collapsible valve body which comprises an impermeable material and placed within the lumen of the neck. In the fully expanded state, the total length of the main tubular body and the transition portion is at least 50 mm, preferably at least 100 mm, more preferably 150 mm, even more preferably 200 mm. The biocompatible material is preferably a metallic substrate selected from the group consisting of titanium, nickel-titanium alloys such as nitinol and Nitinol-DFT®-Platinum, any type of stainless steels, or a cobalt-chromium-nickel alloys such as Phynox®.

The self-expandable braided framework comprises a plurality of layers of wires made of biocompatible material, each layer forming a mesh, the meshes forming a lattice with a plurality of wires of given layers. When observed normal to a wall of the self-expandable braided framework, the lattice defines polygonal opening units. A mean diameter of an inscribed circle of the polygonal opening units is preferably, in fully expanded state, at least 50 μm and at most 200 μm, more preferably at least 100 μm and at most 150 μm.

A ratio ($T_{20}/\emptyset_{25}$) of the thickness ($T_{20}$) of a wall of the self-expandable braided framework to the diameter ($\emptyset_{25}$) of wire is higher than 2.0, preferably at least 3.5, more preferably at least 5.5, even more preferably at least 6.5, still even more preferably at least 7.5. The meshes are, preferably, interlocked forming a lattice with a plurality of wires of given layers, the wires being integrated in the mesh of at least one of the adjacent layers such that meshes of adjacent layers of the framework are substantially offset.

Advantageously, the self-expandable braided framework consists of at least 150 wires, more preferably at least 180 wires, even more preferably at least 250 wires, still even more preferably at least 300 wires. The diameter of wire is preferably at least 30 μm and at most 180 μm, more preferably at least 50 μm and at most 150 μm, even more preferably at least 75 μm and at most 100 μm.

In a fully expanded state, a surface coverage ratio (SCR) of said self-expandable braided framework is preferably at least 35 %, more preferably at least 45%, even more preferably at least 55%, still even more preferably at least 65% and less than 90%.

When the implantable endoluminal prosthesis is deployed in a curved lumen having a H/W ratio between 0.5 and 0.9, the mean diameter ($\emptyset_{IC}$ of inscribed circle (IC) of the polygonal opening units is preferably at least 50 μm and at most 250 μm, a length-related compression ratio (LCR) being between 15% and 40%, and the surface coverage ratio (SCR) of the braided framework being more than 35% at the side of outer curve.

According to a preferable embodiment, the transition portion has a cross-section with a diameter larger than the one of the main tubular body so as to form a globular shape.

According to another preferable embodiment, the self-expandable braided framework further comprises a sealing portion between the proximal end of the braided frame work and the neck, the diameter of sealing portion increasing toward the proximal end of the braided framework. The self-expandable braided framework preferably further comprises an enlarged portion between the distal end of the self-expandable braided framework and the main tubular body, the diameter of enlarged portion increasing toward the distal end of the self-expandable braided framework.

According to another preferable embodiment, the surface of said wires is covered with a phosphonate, preferably gem-bisphosphonate. At least one phosphonate moiety of said gem-bisphosphonate is covalently and directly bonded to the external surface of the wire. The bisphosphonate covers at least 50% of the external surface of the wires as monolayer and as an outermost layer and covers at least 50% of the external surface of the wires as monolayer and as an outermost layer. Advantageously, said gem-bisphosphonate is selected from a group consisting of etidronic acid, alendronic acid, clodronic acid, pamidronic acid, tiludronic acid, risedronic acid or a derivative thereof.

As another embodiment, the surface of said wires are coated with phosphonate containing a hydrocarbon chain comprising 3 to 16 carbon atoms as a linier chain. The phosphorus atom of the phosphonate bonds to the hydrocarbon chain at the alpha-position. Said hydrocarbon chain is further functionalized at its terminal position by a carboxylic group, a phosphonic group or a hydroxyl group. The phosphonate is covalently and directly bonded to the external surface of the wire and covers at least 50% of the external surface of the wires as monolayer and as an outermost layer.

Another subject of the present invention relates to the implantable endoluminal prosthesis described above for use in prevention of embolic stroke for patients during and after prosthetic valves implantation, by placing said implantable endoluminal prosthesis orifices of the coronaries and the supra aortic branches which carries blood to the heart and the brain.

Still another subject of the present invention relates to the implantable endoluminal prosthesis described above for use in improving perfusion of an organ by placing said implantable endoluminal prosthesis in the aorta while covering the orifices of the coronaries and the supra aortic branches which carries blood to the heart and the brain.

## BRIEF DESCRIPTION OF THE FIGURES

Other particularities and advantages of the invention will be developed hereinafter, reference being made to the appended drawing wherein:

FIG. 1 is a side view of a device according to the invention placed in the ventricle of the heart and in the ascending aorta, the arch and the descending aorta;

FIG. 2a is a side view of the device of Fig.1 in fully expanded state;

FIGs. 2b and 2c are cross-views of the device of Fig.2a, respectively with closed and open heart valve;

FIG. 3 is a side view of another embodiment of the device of the invention in fully expanded state;

FIGs. 4a and 4b are perspective views of the tissues forming the valve body;

FIGs. 5 and 6 are side views of other embodiment of the device of the invention in fully expanded state;

FIG. 7 is a cut view of a detail of another embodiment of the device of the invention;

FIG. 8 is a cut view of another embodiment of the device of the invention placed in the ventricle of the heart and in the ascending aorta;

FIG. 9 is a side view of another embodiment of the device of the invention in fully expanded state;

FIG. 10 is a cut in situ view of the embodiment of Fig 9 placed in the ventricle of the heart and in the ascending aorta;

FIG. 11 is a side view of a tubular body deployed in a curved lumen;

FIGs. 12 and 13 are perspective views of the device of the invention, respectively in straight fully expanded state and in deployed state in a curved lumen;

FIGs. 12a and 13a are enlarged views of a detail of respectively Figs 12 and 13;

FIG. 14 is a schematic cross-section view of the aorta showing how to measure the width and height of the aortic arch;

FIG.15 is a schematic magnified view of a portion of an (or another) endoluminal prosthesis according to the present invention;

FIG. 16 is a side view of the endoluminal prosthesis in expanded state;

FIG. 16a is a schematic magnified view of a portion of the endoluminal prosthesis illustrated in FIG. 16;

FIGs. 17a - 17c are a schematic elevation view of a portion of the endoluminal prosthesis with its first layer, the first and second layers, and the first, second and third layers, respectively, showing how to block an embolic material which is trying to go through a wall of the endoluminal prosthesis in front of a coronary's or an aortic branch's inlet;

FIGs. 18a - 18c are a schematic perspective view of the portion of the endoluminal prosthesis shown in FIGs. 17a - 17c, respectively;

FIG. 19a shows a conventional single-layer braided filer device in a fully expanded state and a magnified view of a portion of the filter device;

FIG. 19b shows a conventional single-layer braided filer device deployed in a curved lumen and a magnified view of a portion of the filter device at the outer side of the curve;

FIG. 20 is a partial, schematic magnified, cross-section view of the aortic arch at the orifice of an aortic branch, showing the deployed endoluminal prosthesis according to the present invention;

FIGs. 21a and 21b are a schematic magnified view illustrated in FIG.20, showing how to an embolic material temporally located in front of an aortic orifice is flushed away during the cardiac cycle;

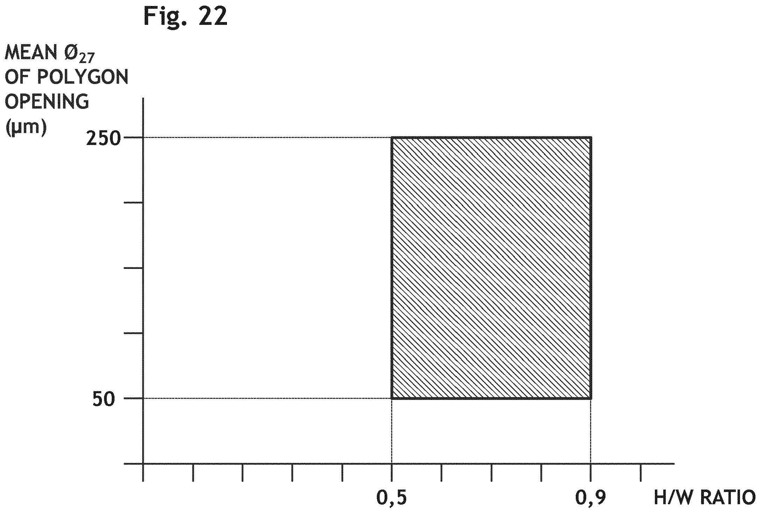

FIG. 22 is a graph representing the relation between (x) the H/W ratio of a curved lumen where an endoluminal prosthesis according to the present invention is deployed, and (y) the mean inscribed circle diameter of mesh opening of the endoluminal prosthesis at the outer side of the curve;

FIG. 23 is a graph representing the relation among (x) the H/W ratio of a curved lumen where an endoluminal prosthesis according to the present invention is deployed, (y) the mean inscribed circle diameter of mesh opening at the outer side of the curve and (z) length-related compression ratio;

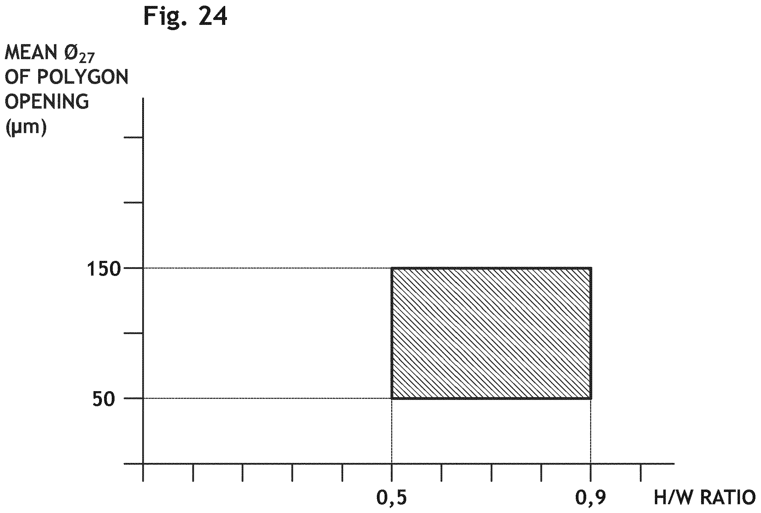

FIG. 24 is a graph representing the relation between (x) the H/W ratio of a curved lumen where an endoluminal prosthesis according to the present invention is deployed, and (y) the mean inscribed circle diameter of mesh opening of the endoluminal prosthesis at the outer side of the curve;

FIG. 25 is a graph representing the relation among (x) the H/W ratio of a curved lumen where an endoluminal prosthesis according to the present invention is deployed, (y) the mean inscribed circle diameter of mesh opening at the outer side of the curve and (z) length-related compression ratio.

## DETAILED DESCRIPTION OF THE INVENTION

As used herein, the term "implantable" refers to an ability of a medical device to be positioned at a location within a body vessel. Implantable medical device can be configured for transient placement within a body vessel during a medical intervention (e.g., seconds, minutes, hours), or to remain in a body vessel permanently.

The terms "endoluminal" or "transluminal" prosthesis refers to a device adapted for placement in a curved or straight body vessel by procedures wherein the prosthesis is advanced within and through the lumen of a body vessel from a remote location to a target site within the body vessel. In vascular procedures, a medical device can typically be introduced "endovascularly" using a catheter over a wire guide under fluoroscopic guidance. The catheters and wire guides may be introduced through conventional access sites in the vascular system.

The term "catheter" refers to a tube that is inserted into a blood vessel to access the target site. In the present description, a "catheter" will designate either a catheter per se, or a catheter with its accessories, meaning needle, guide wire, introducer sheath and other common suitable medical devices known by the man skilled in the art.

The term "preventing" includes rejecting or inhibiting the embolic material from entering a specified blood vessel, such as a branch blood vessel.

To avoid any confusion, in the description herein below, the terms of "opening", "pore" and "window" have their ordinary meaning and are also used interchangeably to refer to an open channel or passageway from one face or surface of a medical device to its other face or surface. Similarly, the terms of "inlet", "junction", "mouth" and "orifice" refer to an area in vasculature where at least one branch blood vessel diverges the main blood vessel.

The term "endothelialisation" refers to a cellular process resulting in ingrowth of endothelial cells onto a device.

The term "permanent" refers to a medical device which may be placed in a blood vessel and will remain in the blood vessel for a long period of time (e.g. months, years) and possibly for the remainder of the patient's life.

The terms "embolus", "embolic material" and "filtrate" refer to a clot or other biologic material which has been brought to its site of lodgement by the blood flow. The obstructing material is most often a blood clot (i.e., thrombus), but may be a fat globule (due to atherosclerosis), piece of tissue or clump of bacteria.

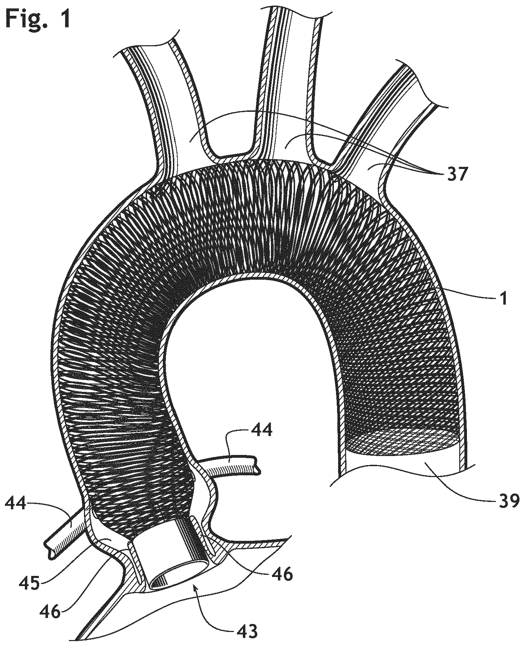

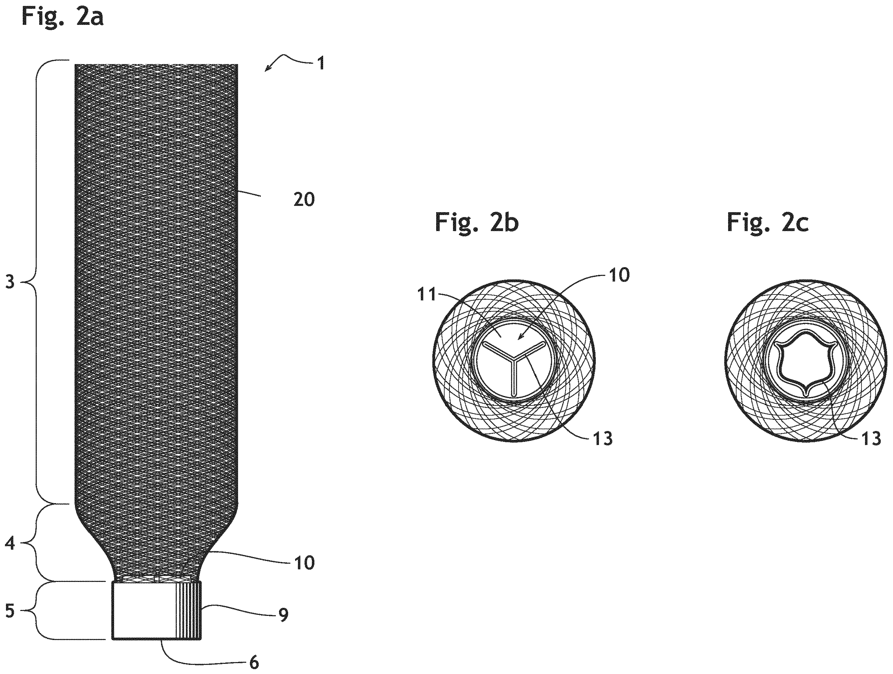

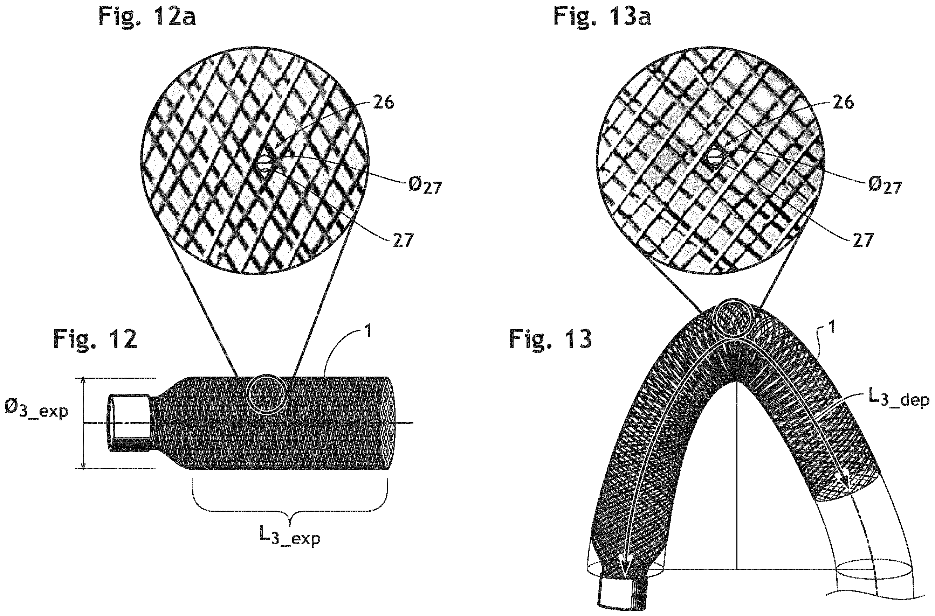

The endoluminal prosthesis **1** is configured to take a compressed shape having a relatively small and relatively uniform diameter when disposed within a delivery system (i.e., "in compressed state"), and to spontaneously take a deployed shape with radially expanded diameter within the delivery location such as a body lumen (i.e., "in deployed state") as shown in FIGs. 1, 8 and 10. As used herein the terms "expanded shape" or "expanded state" refer to a shape or state resulting from the self-expanding properties of a self-spring-back object (e.g., braided framework **20**) when it is allowed to expand without any outer compression force (i.e., non-constricted state) as for example shown in FIGs. 2a to 2c, 3, 5, 6 and 9. Beside these definitions, the term "nominal diameter" designates the diameter of the stent-filter when placed in the targeted vessel. Generally, the nominal diameter ($\varnothing_{nor}$) of a self-expandable device designed to be placed permanently inside a body lumen is 10 to 25% smaller than the external diameter of said device when deployed without external compression force ($\varnothing_{exp}$). Since the diameter ($\varnothing_{39}$) of an aorta **39** is generally between 20 mm and 40 mm, the main tubular body **3** of the self-expandable braided framework **20** is accordingly designed and/or manufactured to have a diameter ($\varnothing_{3\_exp}$) between 22 mm and 50 mm in expanded state. Variations of the diameter of the prosthesis influence, in turn, its length. As shown in FIGs. 12 and 13, the length ($L_{3\_dep}$) of the main tubular body **3** of the invention in deployed state is thus larger than its length ($L_{3\_exp}$) in expanded state. The length-related compression ratio **(LCR)** of the main tubular body **3** can be defined by the relation:

$$LCR = (L_{3\_dep} - L_{3\_exp})/L_{3\_exp}$$

FIG. 1 represents an implantable endoluminal prosthesis **1** according to the present invention deployed within the aorta **39,** particularly from the aortic annulus **43** to the descending aorta and the arch which covers the coronaries **44** and the supra aortic branches **37.**

The implantable endoluminal prosthesis **1** according to the present invention comprises a self-expandable braided framework **20** able to expand from a radially compressed state in a delivery configuration to a radially expanded state and a radially collapsible valve body **10** made of an impermeable material, as shown FIGs. 2a to 2c.

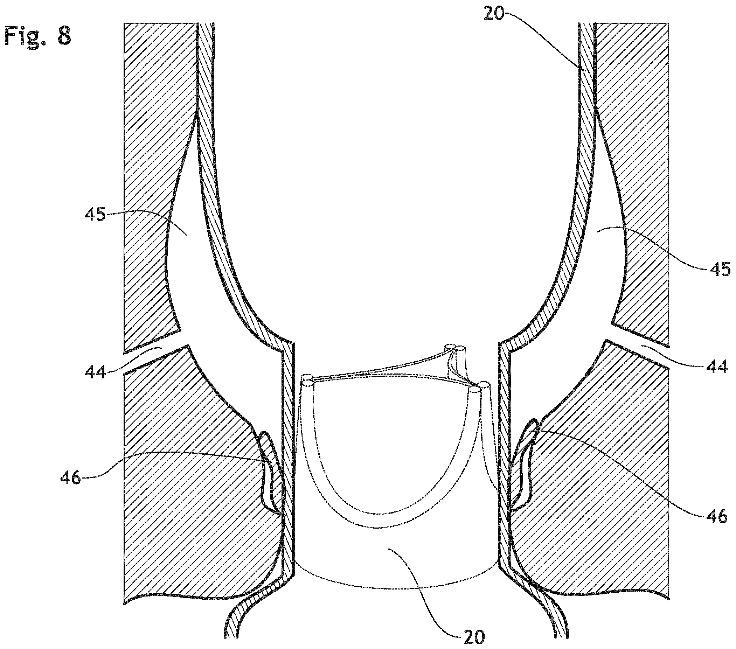

The braided framework **20** has a proximal end **6** configured to extend toward the heart and a distal end **7** configured to extent toward away from the heart. The braided framework **20** comprises a main tubular body **3** comprising a lumen in a cylindrical form with a circular cross-section and a constant diameter at the distal end of the braided framework, a neck **5** comprising a lumen of cylindrical form with a circular cross-section and a constant diameter smaller than the one of said main tubular body **3** at the proximal end of the braided framework **20**, and a transition portion **4** extending between the proximal end of the main tubular body **3** and the distal end of the neck **5**. Said main tubular body **3**, said neck **5** and said transition portion **4** consist of an integrated continuous structure made of a multilayer braid and devoid of any impermeable cover layer. The radially collapsible valve body **10** is placed within the lumen of the neck **5**. In the fully expanded state, the total length of the main tubular body **3** and the transition portion **4** is at least 50 mm so that the wall of the braided framework **20** completely covers the aortic sinus **45** so as to prevent possible embolic material generated in the left ventricle from entering coronaries branched to the aortic sinus **45** as shown in FIG.8. The aortic sinus 45 is the anatomic dilations of the ascending aorta occurring just above the aortic valve **46**.

The total length of the main tubular body **3** and the transition portion **4** is, preferably, at least 100 mm in fully expanded state in order to ensure sealing of the aortic sinus **45** with the self-expandable braided framework **20**. The total length is more preferably at least 150 mm, even more preferably at least 200 mm (still in fully expanded state as shown in FIG.1), so that the braided framework can further cover the supra aortic branches in order to deviate the embolic materials formed not only in the left ventricle but also in the aortic sinus around the aortic valve and the ascending aorta.

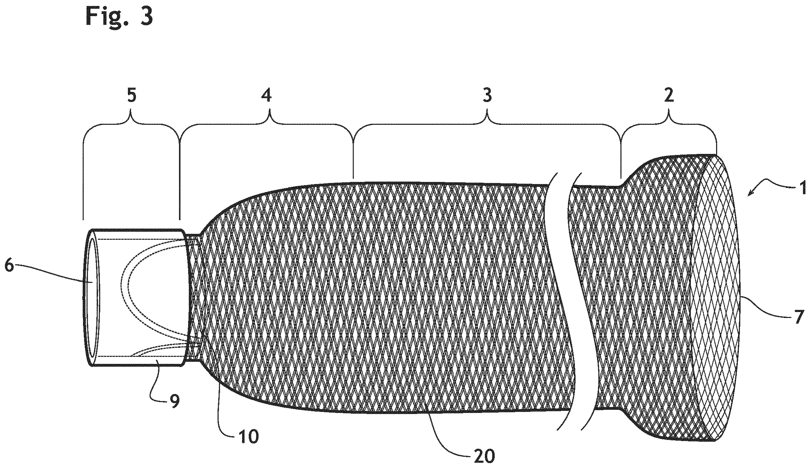

As a preferred embodiment, the self-expandable braided framework **20** further comprises an enlarged portion **2** between the distal end **7** of the braided framework **20** and the main tubular body **3** as illustrated in FIG. 3. The diameter of the enlarged portion **2** increases toward the distal end **7** of the braided framework **20**. The enlarged portion **2** also reduce the risk of a device migration and endoleak after implantation.

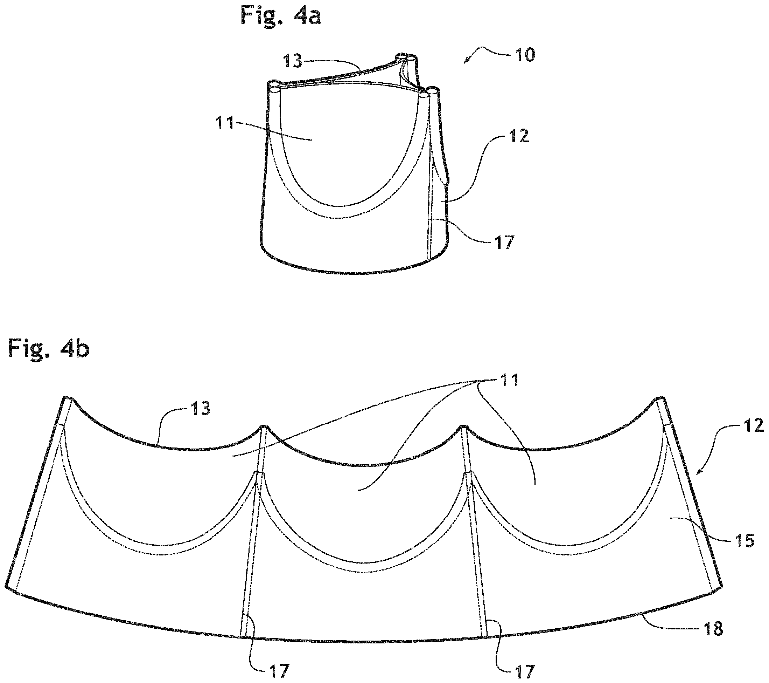

FIGs. 4a and 4b show in a more detailed manner the radially collapsible valve body **10** of the present invention. This valve body comprises a skirt **12** and leaflets **11** which are made of impermeable material. Said skirt **12** and leaflets **11** are preferably cut from a sheet of animal pericardial tissue, such as porcine pericardial tissue, or from another suitable synthetic or polymeric material. The pericardial tissue may be processed in accordance with tissue processing techniques that are per se known in the art of forming and treating tissue valve material. Leaflet **11** has a free edge **13** and a leaflet body **14**. Free edge **13** forms coaptation edge **13** of the finished valve body **10**. Leaflet body **14** is joined to a skirt **12**. Skirt **12** is preferably constructed from the same material as leaflets **11**, and comprises concaved portions **15**, reinforcing areas **17**, and a proximal portion **18**. Each concaved portion **15** is joined to a leaflet body **14** of a respective leaflet **11** by sutures or gluing. The valve body **10** is a truncated cone shape having an axis parallel to the one of the braided framework **20** and preferably comprises a reinforcing means, such as overlapped valve body material, metallic wire and plastic bar that are for example affixed to a wall of the skirt **12** between concaved portions **15** along the axis. This prevents the valve body **10** from turning inside out during the cardiac cycle and/or from migration of valve body placed in the braided framework. The proximal portion **18** of skirt **12** is preferably affixed to an inner wall of the proximal end **6** of the braided framework **20** with attaching means such as sutures and gluing.

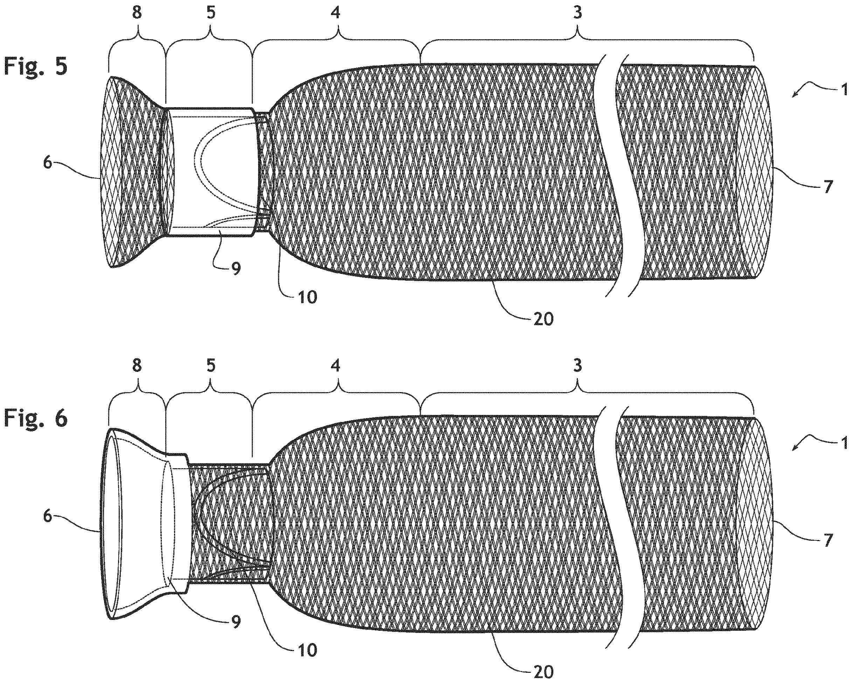

According to another embodiment, illustrated in FIGs. 5 and 6 the self-expandable braided framework **20** further comprises a sealing portion **8** between the proximal end **6** of the braided framework **20** and the neck **5**. The diameter of the sealing portion **8** increases toward the proximal end **6** of the braided framework. The sealing portion **8** also reduces the risk of migration of the device away from the valve site after implantation.

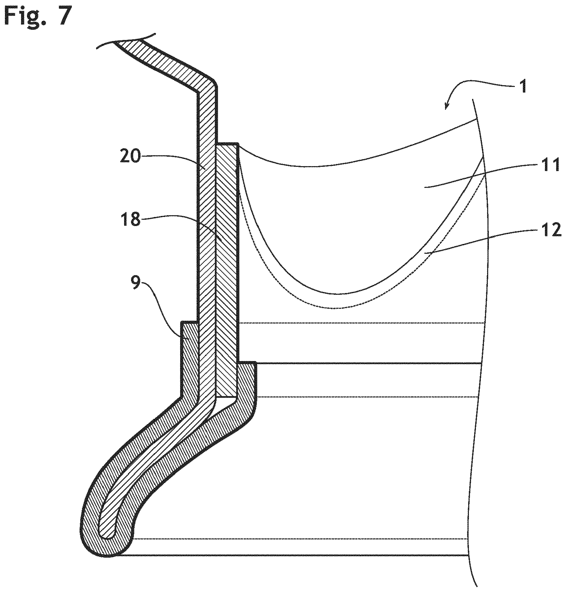

In order to ensure sealing of the aortic annulus **43** and prevent the blood flow from regurgitation, an impermeable biocompatible sleeve **9** can be used to clamp together both proximal ends, **18** and **6,** of skirt **12** and braided framework **20** and affixed by attaching means such as sutures and gluing as illustrated in FIG. 7. This also reduces the risk that wired edges of the proximal end **6** hurt the tissue of aortic annulus **43** when it is deployed in the body. Preferably, the impermeable biocompatible sheet **9** is elastic to accommodate to the change in the length and diameter of the braided framework between its delivery and deployed states.

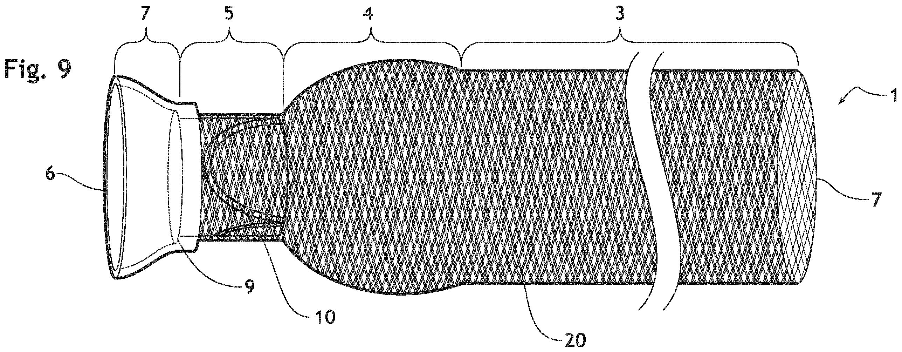

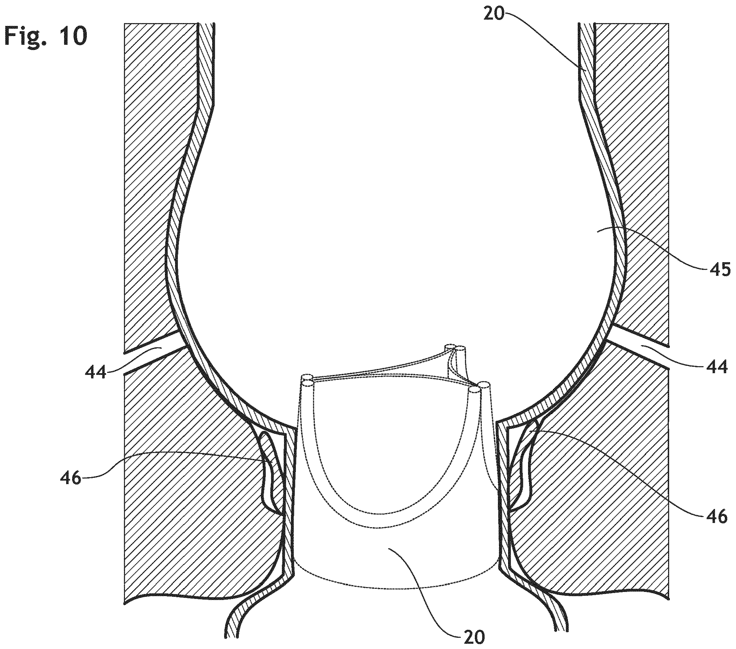

FIG.9 illustrates another embodiment of the present invention, the transition portion **4** has here a cross-section with a diameter larger than the one of the main tubular body **3** so as to form a globular shape. Thanks to this globular configuration, the implantable endoluminal device **1** is well fit to the aortic sinus **45**. As a result, the mouths of the coronaries **44** located in the aortic sinus **45** are well covered **by** the multi-layered wall of the braided framework **20** so that embolic materials formed on the original heart valve **46** or in the aortic sinus **45** are prevented from entering and occluding the coronaries **44,** as shown in FIG.10.

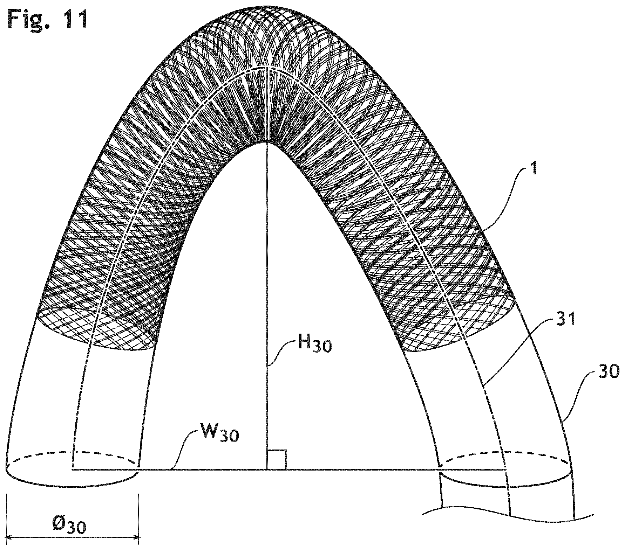

When the tubular body 2 is deployed in a curved lumen **30** as shown in FIG. 11, its length ($L_{3-dep}$) is measured along the midpoint **31** of the curve as indicated in FIG. 13.

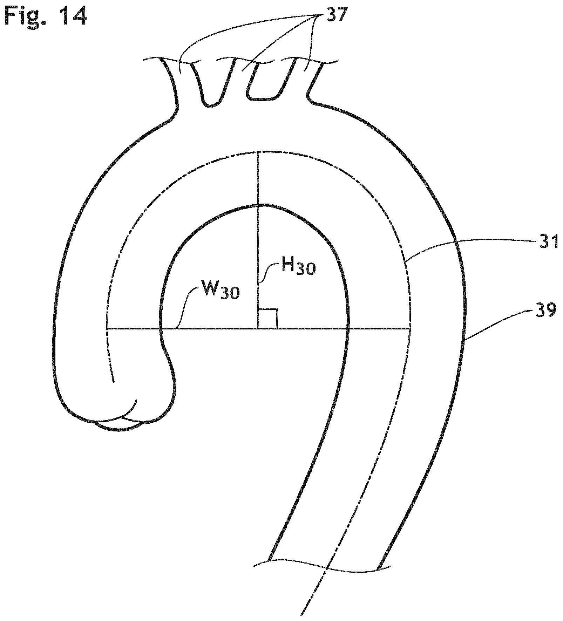

The curve of the aortic arch **39** is generally defined by measuring the width ($W_{39}$) and height ($H_{39}$) of the curve as described by Ou et al. in J. Thrac. Cardiovasc. Surg. 2006; 132: 1105-1111. Width ($W_{39}$) is measured as the maximal horizontal distance between the midpoints **31** of the ascending and descending aorta **39** close to the axial plane going through the right pulmonary artery (RPA); and height ($H_{39}$) of the aortic arch is measured maximal vertical distance between ($W_{39}$) and the highest midpoint **31** of the aortic arch **39** as depicted in FIG. 14. The ratio $H_{39}/W_{39}$ is generally in a range of 0.5 and 0.9. For example, when the value is 0.9 (the worst scenario), the aortic arch is extremely acute as depicted in FIG. 11. This can cause a kinking of previously described "conventional" filters, which have poor hoop force. Furthermore, one will notice the difference of mesh opening between its straight form greater in comparison with the one deployed in a curve having about 0.6 of the H/W ratio (which is usually observed in healthy aortas). One of the advantages of the present invention is that the mesh windows are not compromised by this extremely acute curve because of the combination of the layers, as is apparent from a comparison between FIGs. 12a and 13a.

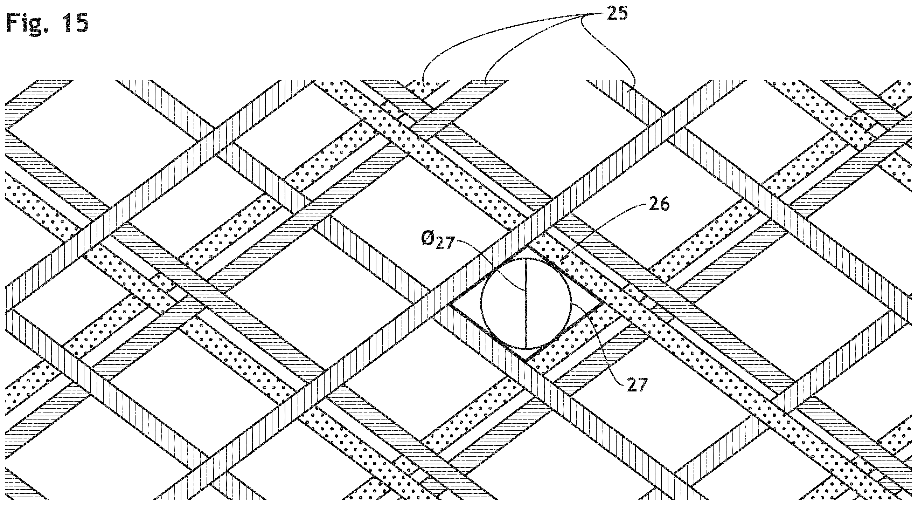

As shown in FIG. 15, the braided framework 20 comprises a plurality of layers 22, 23, 24 of wires 25 made of biocompatible material. The wires preferably have a diameter ($\varnothing_{25}$) of at least 30 $\mu$m, preferably at least 50 $\mu$m and at most 180 $\mu$m, more preferably at least 75 $\mu$m and at most 150 $\mu$m, even more preferably at most 100 $\mu$m. Each layer of the braided framework 20 forms a mesh. When observed normal with respect to a wall, meshes of the braided frame 20 form a lattices with a plurality of level of wires 25. Preferably, the meshes are interlocked with each other so as to form an interlocked multi-layer structure. The term "interlocked multi-layer" refers to a framework comprising multiple layers, 22, 23, 24, whose plies are not distinct at the time of braiding, for example a given number of wires of the plies 22a of the first layer 22 being interlocked with the plies 23a of the second layer 23 and/or other layers 24. Said interlocked multi-layer, for example, can be formed by using the braiding machine described in EP1248372. The braided framework 20 of the endoluminal prosthesis 1 is made of preferably at least 150 of wires 25, more preferably at least 180 wires, even more preferably at least 250 wires, still even more preferably at least 300 wires, and preferably at most 600 wires.

The lattice defines opening units 26 having a polygonal shape defined by sides (i.e. wire segments). The polygonal shape is preferably quadrangle, more preferably parallelogram. "Parallelogram" means a simple quadrilateral with two pairs of parallel sides; the facing sides of a parallelogram are of equal length; the opposite angles of a parallelogram are of equal measure; and the diagonals bisect each other. Parallelograms include squares, rectangles, and lozenges. As used herein, "inscribed circle" 27 refers to the largest circle that can be drawn inside the polygonal opening unit 26 and tangent to a maximum of its sides (i.e. wires segments 25) as depicted in FIGs. 12a, 13a and 15.

The size of inscribed circle 27 directly reflects the efficacy to deflect embolic material 35, particularly microembolus that would have flown into the supra aortic branches, to the descending aorta. "Microembolus" refers to an embolus of microscopic size, for example, a tiny blood clot or a little clump of bacteria. Micro-emboli are either gaseous or solid embolic material. The gaseous micro-emboli can originate from mechanically induced cavitation created by a prosthetic heart valve. They have an approximate diameter of 4 $\mu$m and cause normally no deleterious effect on the brain. In contrast solid microemboli are much bigger than gaseous micoremboli, having an approximate diameter of 100 $\mu$m. The larger size of solid microemboli compared to the size of capillaries (diameter 7 - 10 $\mu$m) can cause blockade of micro circulation. In J. Endovasc. Ther, 2009; 16; 161-167, "Reduction of cerebral embolixation in carotid angioplasty: An in-vitro experiment comparing 2 cerebral protection devices" published by Charalambous et. al., either gaseous or small emboli having diameter less than 200 $\mu$m cause only clinically unperceived cerebral ischemia.

Therefore, in order to reroute embolic material having more than 200 $\mu$m, a mean diameter ($\varnothing_{27}$) of inscribed circle 27 (IC) of polygonal openings 26 is preferably at most 200 $\mu$m in a curved deployed configuration to comply to the aortic arch geometry, preferably at most 150 $\mu$m. At the same time, since the openings should be large enough to let the blood components get through the wall of the prosthesis 1 and keep adequate perfusion, the mean IC should be at least 50 $\mu$m, preferably at least 75 $\mu$m, more preferably at least 100 $\mu$m. The mean diameter ($\varnothing_{27}$) of inscribed circle 27 (IC) of polygonal openings 26 means the value found by adding together all the diameters of inscribed circle 27 and dividing the total by the total number of openings 26.

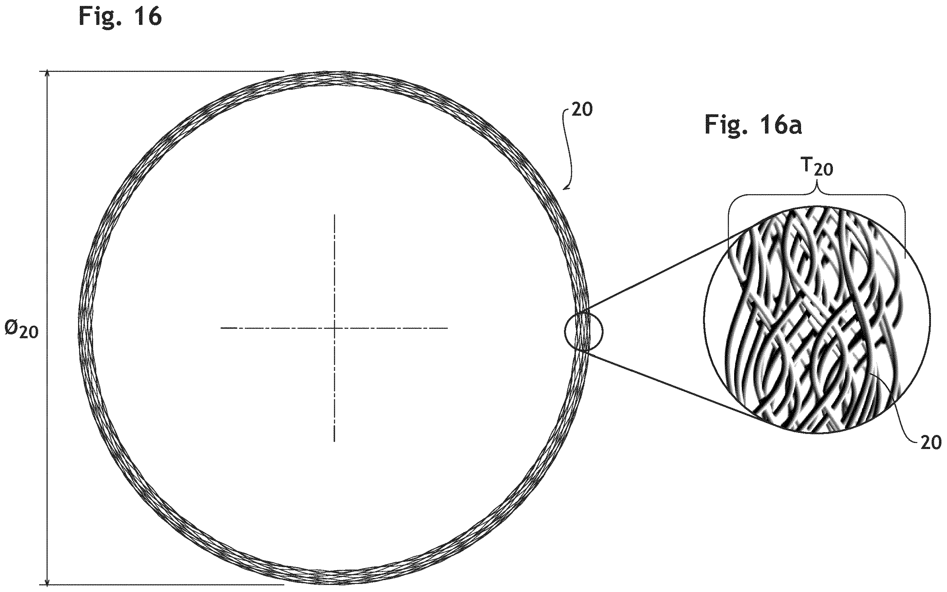

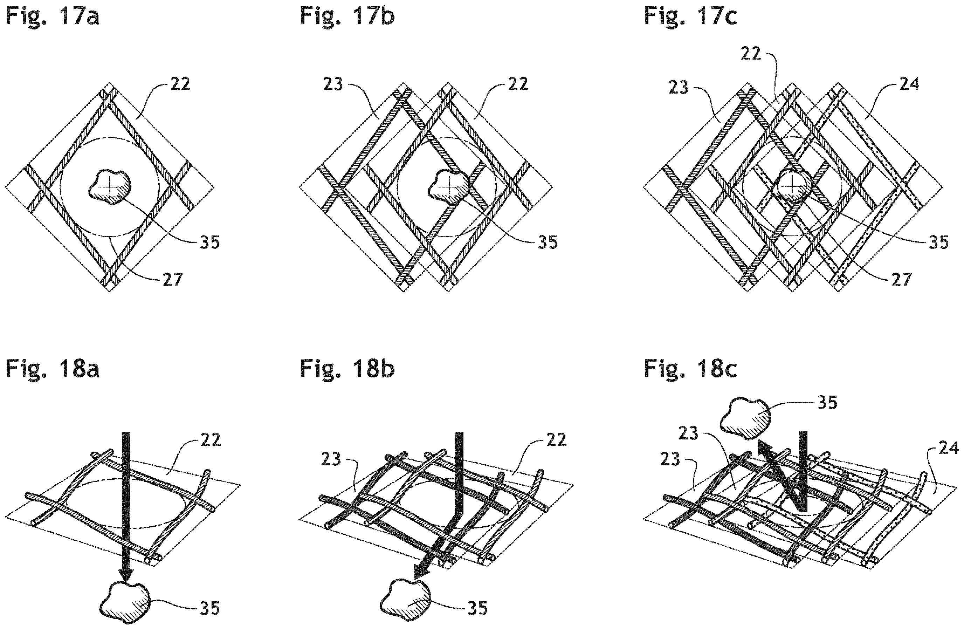

One of advantages of the present invention is that the braided framework 20, having higher value of the ratio $T_{20}/\varnothing_{25}$, can effectively prevent an embolic material 35 from going through its wall as shown in FIGs. 17a-17c, 18a-18c and 21b in comparison with a conventional filter having a $T_{20}/\varnothing_{25}$ ratio less than 2.0. The ratio ($T_{20}/\varnothing_{25}$) of the wall thickness ($T_{20}$) of the braided framework 20 to the wire diameter ($\varnothing_{25}$) being more than 2.0 characterizes the braided framework having more than a single layer of mesh. The greater the ratio $T_{20}/\varnothing_{25}$, the more layers the braided framework 20 will comprise. Each wire forming multiple-layers aligned substantially parallel in the wall, as shown in FIG. 16, has a chance to deviate or block an embolic material trying to get through the wall of the endoluminal prosthesis 1 as schematically explained in FIGs. 17a - 17c and 18a - 18c, the present structure can thus increase the emboli rerouting efficacy.

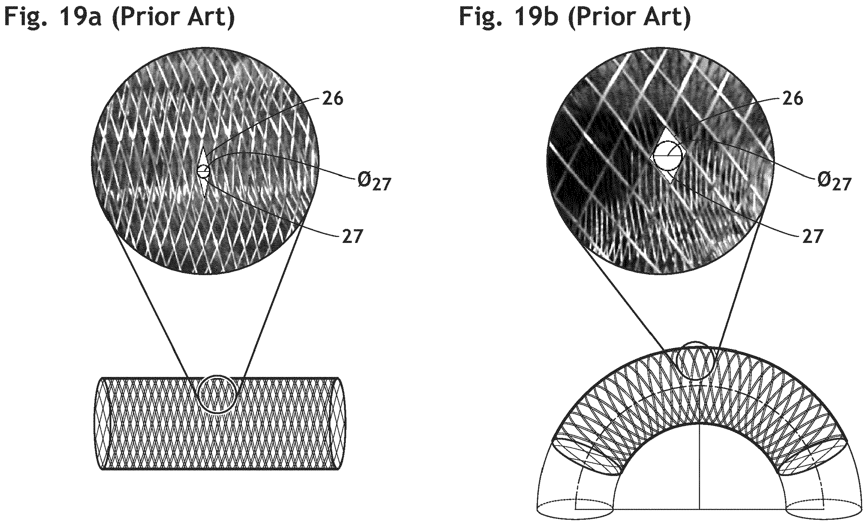

Furthermore, interlocked multiple-layer configuration having a ratio $T_{20}/\varnothing_{25}$ higher than 3.5 brings about an important technical property: when it is deployed in a curved lumen having an H/W ratio between 0.5 and 0.9, the mean inscribed circle diameter ($\varnothing_{27}$) of opening units being at least 50 $\mu$m and at most 250 $\mu$m keep its desirable opening size, even at the outer side of the curve 29 as defined in FIGs 11 and 14. Since the mouths of the supra aortic branches are located at the outer side of the arch, it is most important to set an optimal opening size at the outer side when deployed in an aortic arch geometry in order to improve filtering efficacy. Wires of the interlocked multiple-layer configuration of the invention shift to keep a regular distance between adjacent parallel; resulting in that the mean inscribe diameter ($\varnothing_{27}$) stays almost the same either in a curved state (as shown in FIGs. 12a and 13a) or in straight configuration. On the Contrary, when a conventional single-layer mesh-like tube having less than 2.0 of $T_{20}/\varnothing_{25}$ is deployed in a curved lumen, the mesh openings at the outer side of the curve are much wider than the mesh openings in a straight configuration as shown in FIGs 19a to 19d. Therefore, the ratio of $T_{20}/\varnothing_{25}$ of the braided framework **20** of the invention should be more than 2.0, preferably at least 3.5, more preferably at least 5.5, even more preferably at least 6.5, still even more preferably 7.5.

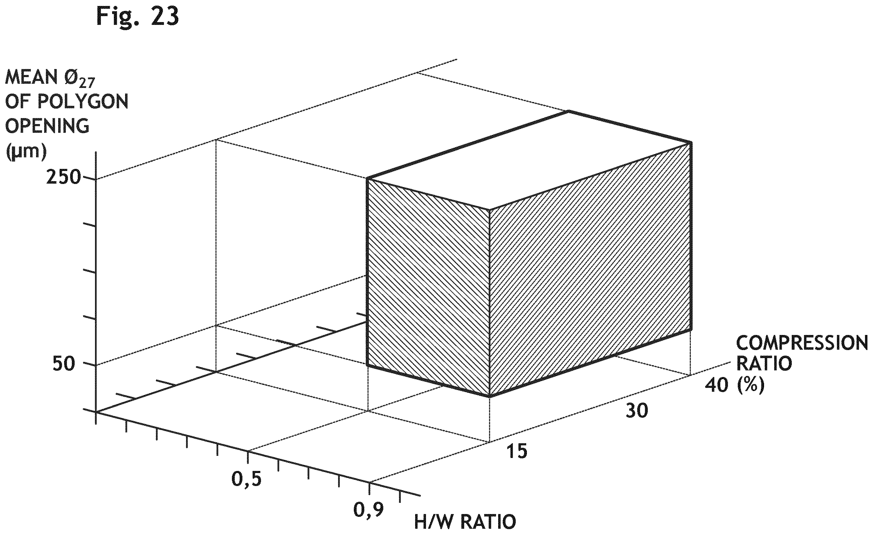

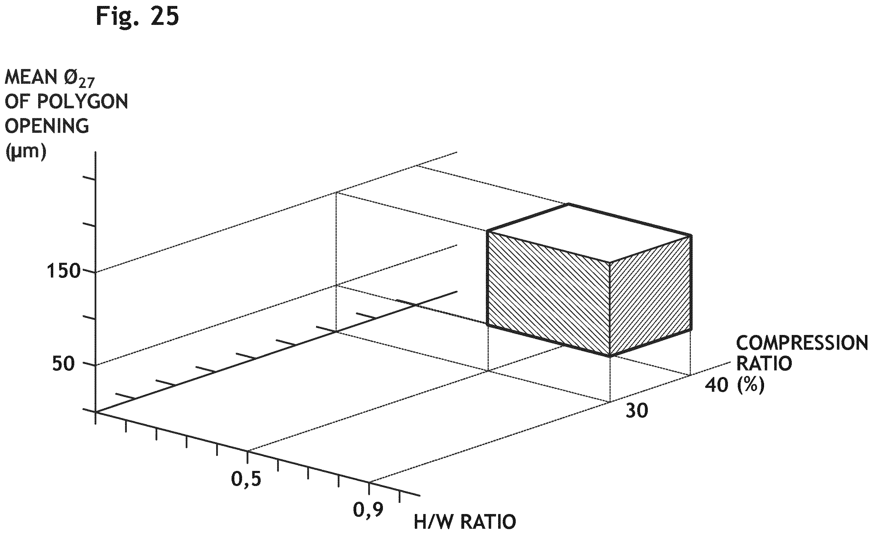

As mentioned above, the aorta exhibits arterial compliance. Hence, an endoluminal prosthesis for aorta should have enough hoop force to deal with the arterial compliance; otherwise it may cause complications such as device migration and kinking. The device migration is an undesired displacement of the device after implantation and kinking is a phenomenon well known to men skilled in the art to occur during stent placement in a curved vessel. In order to obtain sufficient hoop force, the length-related compression ratio **(LCR)** should be in a range of 15% and 40%, preferably 30% and 40%. The relations of **LCR** to the H/W ratio and the mean inscribed circle diameter ($\varnothing_{27}$) according to the present invention are shown in FIGs. 23 and 25.

The surface coverage ratio **(SCR)** of the braided framework **20** is defined by the relation:

$$SCR = S_w/S_t$$

wherein: "$S_w$" is the actual surface covered by wires **25** composing the braided framework **20,** and **"$S_t$"** is the total surface area of the wall of the braided framework **20.** In a fully expanded state, **SCR** of the braided framework **20** is preferably at least 45%, more preferably more than 50%, even more preferably at least 55%, still even more preferably at least 65% and at most 90%. When deployed in a C-curved lumen **30** having a nominal diameter of the endoluminal prosthesis **1** and the $H_{30}/W_{30}$ ratio between 0.5 and 0.9, the braided framework **20** with at least 3.5 of the ratio of $T_1/\varnothing_{25}$ (preferably 5.5, more preferably at least 6.5, even more preferably at least 7.5) can provide almost same surface coverage ratio **(SCR)** along its outer curve **29** as the one in its straight configuration, i.e. at least 35%. It is another advantage of the present invention, resulting in improvement of emboli rerouting efficacy.

Filtering devices known in the art often become clogged and need to be cleaned or even replaced. An endoluminal prosthesis designed to be positioned permanently in a blood vessel should have an inherent ability to clean itself or be cleaned by endogenous forces or effect so as to avoid periodic cleaning by a physician or removal of the device from the blood vessel.

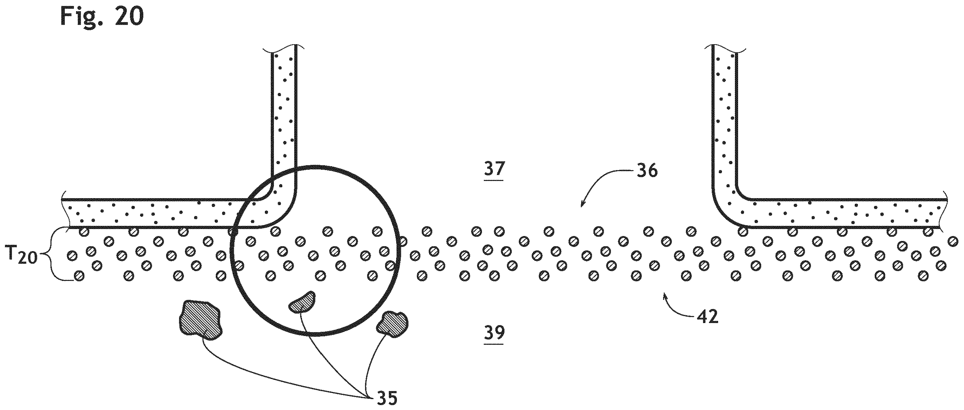

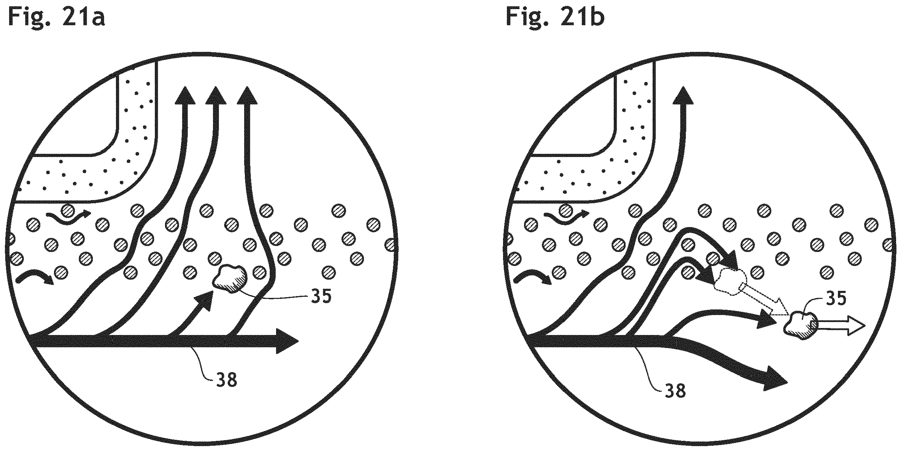

The self-expandable braided framework **20** of endoluminal prosthesis **1** having a sufficient wall thickness ($T_{20}$) against the size of the opening **26,** i.e. the inscribed circle diameter ($\varnothing_{27}$), imparts higher self-cleaning property when compared with conventional filter devices. As shown in FIGs. 20, 21a and 21b, some embolic materials **35** flowing about an orifice **36** of supra aortic branch **37** are temporally pushed against an interior surface **42** of the self-expandable braided framework **20** in front of the supra aortic branches **37** as a result of blood inflow through a wall thereof during the ventricular systole and the relaxation phase of the cardiac cycle. Thanks to the sufficient wall thickness $T_{20}$ of the braided framework **26,** these embolic materials **35** are kept trapped on the interior surface **42** instead of passing through the wall, and are then flushed away, back into the aortic blood stream **38** during the atria systole, as a result of the flushing expelling force. The term "flushing expelling force" refers to an inherent property of the present prosthesis. Specifically, it is the force that is applied to the embolic material **35** by the flowing aortic blood **38** with which it comes in contact.

Studies and experiments carried by the inventor led to surprising and unexpected conclusions. If the ratio $T_{20}/\varnothing_{25}$ is smaller than 2.0 as in conventional filters, the embolic material **35** is either flushed through the mesh openings and enters into the arterial branches or accumulates till it blocks the blood flow at the mouths of the branches. The greater the ratio $T_{20}/\varnothing_{25}$, the greater the flushing expelling force the self-expandable braided framework **20** will exhibits. Therefore, the present endoluminal prosthesis **1** having a ratio $T_{20}/\varnothing_{25}$ higher than 2.0, preferably of at least 3.5, more preferably of at least 5.5, even more preferably of at least 6.5, reduces the occlusion risk of the branches mouths covered thereby, resulting in an increase of safety in use.

The biocompatible material used in the invention is preferably a metallic substrate selected from a group consisting of stainless steels (e.g., 316, 316L or 304); nickel-titanium alloys including shape memory or superelastic types (e.g., nitinol, Nitinol-DFT®-Platinum); cobalt-chrome alloys (e.g., elgiloy); cobalt-chromium-nickel alloys (e.g., phynox); alloys of cobalt, nickel, chromium and molybdenum (e.g., MP35N or MP20N); cobalt-chromium-vanadium alloys; cobalt-chromium-tungsten alloys; magnesium alloys; titanium alloys (e.g., TiC, TiN); tantalum alloys (e.g., TaC, TaN); L605;. Said metallic substrate is preferably selected from the group consisting of titanium, nickel-titanium alloys such as nitinol and Nitinol-DFT®-Platinum, any type of stainless steels, or a cobalt-chromium-nickel alloys such as Phynox®.

As additional surprising effect, the perfusion in the branches is improved in accordance with the increase of the ratio $T_{20}/\varnothing_{25}$. "Perfusion" is, in physiology, the process of a body delivering blood to capillary bed in its biological tissue. The terms "hypoperfusion" and "hyperperfusion" measure the perfusion level relative to a tissue's current need to meet its metabolic needs. Since the implantable medical device of the invention increases the perfusion in the <u>supra</u> aortic branches it covers, the function**ing** of the organs to which the supra aortic branches carries the blood is improved.

As indicated in US Patent Application No. US2006/0015138, it is known that coatings preferred for blood filtering means should be highly hydrophobic [as are for example polytetraethylfluorine (PTFE), polyvinylfluoridene (PVDF), and polyalilene] so as to decrease the degree of friction between the blood and the surface of the device and enhance the blood inflow to branches.

Surprisingly, the inventor discovered that when combining the braided framework **20** of his invention with a coating of a phosphorous-based acid formed on the braided framework **20** and attached permanently via covalent bound to the surface of braided wire **25,** he obtained an interesting surface mechanical property that improved embolic rerouting efficacy while keeping an adequate permeability of the braided framework **20** at portions on orifices of <u>supra</u> aortic branches. The phosphorousbased acid used can be selected from organic phosphonic acids having the formula $H_2R^1PO_3$ wherein $R^1$ is an organic ligand with a carbon atom directly bonded to phosphorus at its alpha-position. At least one phosphonate moiety is covalently and directly bonded to the external surface of the metallic substrate in the coating. In one preferred embodiment, said organic ligand comprises a hydrocarbon chain with between 3 and 16 carbon atoms. The organic ligand is further functionalized at its terminal carbon (i.e. at the opposite end of the alphaposition) so as to increase an interaction between the coating and the embolic material **35** flowing in an aorta. Said functional groups may be a hydroxyl group, a carboxylic group, an amino group, a thiol group, phosphonic group or chemical derivatives thereof. Preferably, the substituent is a carboxylic group, phosphonic group or hydroxyl groups. Said coatings provide improved embolic rerouting efficacy while promoting endothelium formation on the interior wall of the implantable medical device covering the artery wall except portions covering branches' orifices, and keeping an adequate permeability of the braided framework at portions in front of supra aortic branches. Preferably, the number of carbon atoms comprised in the organic ligand is at least 6 and at most 16 as a linier chain, more preferably at least 8 and at most 12. Said phosphonic acid may be selected from a group consisting of 6-phosphonohexanoic acid, 11-phosphonoundecanoic acid, 16-phosphonohexadecanoic acid, 1,8-octanediphosphonic acid, 1,10-decyldiphosphonic acid and (12-phosphonododecyl)phosphonic acid. One of carbon atoms, $-(CH_2)-$, of the organic ligand may be substituted by a tertiary amino group, $-N(R^2Y)-$. The substituent of tertiary amino group has an alkyl group, $-R^2Y$, the terminal carbon of which is functionalized by carboxylic acid, phosphonic acid or a derivative thereof. Said phosphonic acid comprising the tertiary amino group is preferably selected from a group consisting of N-(phosphonomethyl)iminodiacetic acid and N,N-bis(phosphonomethyl) glycine). In another preferred embodiment, the phosphonic acid may be further functionalized at the alpha-position of the organic ligand by a supplementary phosphonic acid and/or hydroxyl group such as 5-hydroxy-5,5'-bis(phosphono)pentanoic acid.

In another preferred embodiment, coatings are formed from germinal bisphosphonates characterized by two C-P bonds located on the same carbon atom defining a P-C-P structure. At least one phosphonate moiety of the bisphosphonate is covalently and directly bonded to the external surface of the metallic substrate in the coating. The bisphosphonate covers at least 50% of the external surface of the metallic substrate as monolayer and as an outermost layer. Preferably $R^3$ represents $-C_{1-16}$alkyl substituted with -COOH or -OH at the terminal position; and $R^4$ represents -OH. Preferably, said gembisphosphonate is etidronic acid, alendronic acid, clodronic acid, pamidronic acid, tiludronic acid, risedronic acid or a derivative thereof.

## Method of Deployment

According to one preferred method, the endoluminal prosthesis **1** of the invention is deployed by using an endoluminal prosthesis delivery apparatus **100.** This apparatus **100** is designed to be driven by an operator from the proximal site on through the vascular system so that the distal end of the apparatus can be brought close to the implantation site, where the prosthesis **1** can be unloaded from the distal end of the apparatus **100.** The delivery apparatus **100** comprises the prosthesis **1** itself, a prosthesis receiving region wherein the prosthesis **100** has been introduced, a central inner shaft and a retracting sheath. Preferably, the apparatus **100** further comprises a self-expanding holding means that is compressed within the sheath, the distal portion of which encircles the proximal potion of the prosthesis, and the proximal end of which is permanently joined to the inner shaft with a joint so as to provide the apparatus **100** with a function of re-sheathing a partially unsheathed prosthesis into a retracting sheath. To deploy the prosthesis **1** at a desired location in **the** aorta, the distal end of the retracting sheath is brought to the aortic annulus and the retracting sheath is progressively withdrawn from over the prosthesis **1** toward the proximal end of the delivery apparatus **100.** Once the sheath is adjacent the proximal end of the holding means, the prosthesis **1** is partially allowed to self-expand to a deployed shape. By continually retracting the sheath proximally, the holding means is released from the sheath and deploys while under the effect of the temperature of the organism and/or because of their inherent elasticity. In order to prevent a prosthesis migration after implantation, an oversized prosthesis **1** is generally chosen which has a diameter in its "nominal" expanded state being 10-40% greater than the diameter of the body lumen at the implantation site. Such prosthesis **1** exerts a sufficient radial force on an inner wall of the body lumen and is thus fixed firmly where it is implanted. Since, upon deployment, the radial force provided by the deployed part of the prosthesis **1** onto the wall of the aorta becomes greater than the grasping force of the deployed holding means in its deployed state, the holding means can release the prosthesis at the deployed position without undesired longitudinal displacement when retracting the inner shaft proximally together with the sheath.

**Claims**

1. Implantable endoluminal prosthesis **(1)** suitable for deployment from the aortic annulus to the aorta comprising:

   1) a self-expandable braided framework **(20)** able to expand from a radially compressed state in a delivery configuration to a radially expanded state, the self-expandable braided framework **(20)** being formed of braided wires having a given diameter ($\varnothing_{25}$) and having a proximal end **(6)** configured to extend toward the heart and a distal end **(7)** configured to extent toward away from the heart and extending along an axis, the self-expandable braided framework **(20)** comprising:

   a) at the distal end **(7)** of the self-expandable braided framework **(20),** a main tubular body **(3)** comprising a lumen in a cylindrical form with a circular cross-section and a constant diameter;
   b) at the proximal end **(6)** of the self-expandable braided framework **(20),** a neck **(5)** comprising a lumen in a cylindrical form with a circular cross-section and a constant diameter smaller than the one of said main tubular body **(3); and**
   c) a transition portion **(4)** extending between the proximal end **(6)** of the main tubular body **(3)** and the distal end of the neck **(5),**

   said main tubular body **(3),** said neck **(5)** and said transition portion **(4)** consisting of an integrated structure being devoid of any impermeable cover layer, and forming a wall having a thickness ($T_{20}$),
   2) a radially collapsible valve body **(10)** comprising an impermeable material placed within the lumen of the neck **(5),**

   **characterized in that**, in the fully expanded state, the total length of the main tubular body **(3)** and the transition portion **(4)** is at least 50 mm, the self-expandable braided framework **(20)** comprising a plurality of layers **(22, 23, 24)** of wires **(25)** made of biocompatible material, each layer forming a mesh, the meshes forming a lattice with a plurality of wires **(25)** of given layers **(22, 23, 24),** the lattice, when observed normal to a wall of the self-expandable braided framework **(20),** defining polygonal opening units **(26),** a ratio ($T_{20}/\varnothing_{25}$) of the thickness ($T_{20}$) of a wall of the self-expandable braided framework **(20)** to the diameter ($\varnothing_{25}$) of wire **(25)** being higher than 2.0.

2. Implantable endoluminal prosthesis **(1)** according to claim 1, wherein the meshes are interlocked forming a lattice with a plurality of wires of given layers, the wires being integrated in the mesh of at least one of the adjacent layers such that meshes of adjacent layers of the framework are substantially offset.

3. Implantable endoluminal prosthesis **(1)** according to either one of claim 1 or 2, wherein, in the fully expanded state, the total length of the main tubular body **(3)** and the transition portion **(4)** is at least 100 mm, preferably 150 mm, more preferably 200 mm.

4. Implantable endoluminal prosthesis **(1)** according to any one of preceding claims, wherein the ratio ($T_{20}/\varnothing_{25}$) is at least 3.5, more preferably 5.5, even more preferably 6.5, still even more preferably 7.5.

5. Implantable endoluminal prosthesis **(1)** according to any one of preceding claims wherein the self-expandable braided framework **(20)** consists of at least 150 wires, more preferably at least 180 wires, even more preferably at least 250 wires, still even more preferably at least 300 wires.

6. Implantable endoluminal prosthesis **(1)** according to any one of preceding claims, wherein the diameter of wire **(25)** is at least 30 $\mu$m and at most 180 $\mu$m, preferably at least 50 $\mu$m and at most 150 $\mu$m, more preferably at least 75 $\mu$m and at most 100 $\mu$m.

7. Implantable endoluminal prosthesis **(1)** according to any one of preceding claims, wherein , in a fully expanded state, a surface coverage ratio (SCR) of said self-expandable braided framework **(20)** is at least 35 %, preferably at least 45%, more preferably at least 55%, even more preferably at least 65% and less than 90%.

8. Implantable endoluminal prosthesis **(1)** according to any one of preceding claims, wherein a mean diameter ($\varnothing_{27}$) of an inscribed circle **(27)** of the polygonal opening units **(26)** is, in fully expanded state, at least 50 $\mu$m and at most 200 $\mu$m, preferably at least 100 $\mu$m and at most 150 $\mu$m.

9. Implantable endoluminal prosthesis **(1)** according to any one of preceding claims, wherein, when the implantable endoluminal prosthesis **(1)** is deployed in a curved lumen having a H/W ratio between 0.5 and 0.9, the mean diameter ($\varnothing_{27}$) of inscribed circle **(27)** of the polygonal opening units **(26)** is at least 50 μm and at most 250 μm, a length-related compression ratio (LCR) being between 15% and 40%, and the surface coverage ratio (SCR) of the self-expandable braided framework **(20)** being more than 35% at the side of outer curve.

10. Implantable endoluminal prosthesis **(1)** according to any one of preceding claims, wherein the transition portion **(4)** has a cross-section with a diameter larger than the one of the main tubular body **(3)** so as to form a globular shape.

11. Implantable endoluminal prosthesis **(1)** according to any one of preceding claims, wherein the self-expandable braided framework **(20)** further comprises a sealing portion **(8)** between the proximal end **(6)** of the braided frame work and the neck **(5),** the diameter of sealing portion **(8)** increasing toward the proximal end **(6)** of the braided framework.

12. Implantable endoluminal prosthesis **(1)** according to any one of preceding claims, wherein the self-expandable braided framework **(20)** further comprises an enlarged portion **(2)** between the distal end **(7)** of the self-expandable braided framework **(20)** and the main tubular body **(3),** the diameter of enlarged portion increasing toward the distal end **(7)** of the self-expandable braided framework **(20).**

13. Implantable endoluminal prosthesis according to any one of any one of preceding claims, wherein the biocompatible material is a metallic substrate selected from the group consisting of titanium, nickel-titanium alloys such as nitinol and Nitinol-DFT®-Platinum, any type of stainless steels, or a cobalt-chromium-nickel alloys such as Phynox®.

14. Implantable endoluminal prosthesis **(1)** according to claim 13, wherein the surface of said wires is covered with a gem-bisphosphonate so that at least one phosphonate moiety is covalently and directly bonded to the external surface of the wire **(25),** and the gem-bisphosphonate covering at least 50% of the external surface of the wires **(25)** as monolayer and as an outermost layer.

15. Implantable endoluminal prosthesis **(1)** according to claim 14, wherein the surface of said wires are coated with phosphonate containing a hydrocarbon chain comprising 3 to 16 carbon atoms as a linier chain, the phosphorus atom of the phosphonate bonding to the hydrocarbon chain at the alpha-position, said hydrocarbon chain being further functionalized at its terminal position by a carboxylic group, a phosphonic group or a hydroxyl group, the phosphonate being covalently and directly bonded to the external surface of the wire **(25)** and covering at least 50% of the external surface of the wires **(25)** as monolayer and as an outermost layer.

16. Implantable endoluminal prosthesis **(1)** according to any one of preceding claims for use in prevention of embolic stroke for patients during and after prosthetic valves implantation, by covering with said implantable endoluminal prosthesis **(1)** orifices of the coronaries and the supra aortic branches which carries blood to the heart and the brain.

17. Implantable endoluminal prosthesis **(1)** according to any one of claims 1 to 9 for use in improving perfusion of an organ by covering with said implantable endoluminal prosthesis (1) orifices of the coronaries and the supra aortic branches which carries blood to the heart and the brain.

**Patentansprüche**

1. Implantierbare endoluminale Prothese **(1),** die zum Einsatz vom Aortenring zur Aorta geeignet ist, umfassend:

1) ein selbstexpandierbares geflochtenes Gerüst **(20),** das von einem radial komprimierten Zustand in einer Einsatzkonfiguration zu einem radial expandierten Zustand expandieren kann, wobei das selbstexpandierbare geflochtene Gerüst **(20)** aus geflochtenen Drähten mit einem gegebenen Durchmesser ($\varnothing_{25}$) gebildet wird und ein proximales Ende **(6),** das dazu ausgelegt ist, sich zum Herz zu erweitern, und ein distales Ende **(7),** das dazu ausgelegt ist, sich vom Herz weg zu erstrecken und sich entlang einer Achse zu erstrecken, aufweist, wobei das selbstexpandierbare geflochtene Gerüst **(20)** Folgendes umfasst:

a) am distalen Ende **(7)** des selbstexpandierbaren geflochtenen Gerüsts **(20)** einen röhrenförmigen Hauptkörper **(3),** der ein Lumen in einer zylindrischen Form mit einem kreisförmigen Querschnitt und einem konstanten Durchmesser umfasst;
b) am proximalen Ende **(6)** des selbstexpandierbaren geflochtenen Gerüsts **(20)** einen Hals **(5),** der ein Lumen in einer zylindrischen Form mit einem kreisförmigen Querschnitt und einem konstanten Durchmesser, der kleiner als der des röhrenförmigen Hauptkörpers **(3)** ist, umfasst; und
c) einen Übergangsabschnitt **(4),** der sich zwischen dem proximalen Ende **(6)** des röhrenförmigen Hauptkörpers **(3)** und dem distalen Ende des Halses **(5)** erstreckt, wobei der röhrenförmige Hauptkörper **(3)**, der Hals **(5)** und der Übergangsabschnitt **(4)** aus einer integrierten Struktur bestehen, die frei von einer undurchlässigen Abdeckschicht ist, und eine Wand mit einer Dicke ($T_{20}$) bilden,

2) einen radial kollabierbaren Ventilkörper **(10)**, der ein undurchlässiges Material umfasst, das im Lumen des Halses **(5)** platziert ist,

**dadurch gekennzeichnet, dass** die Gesamtlänge des röhrenförmigen Hauptkörpers **(3)** und des Übergangsabschnitts **(4)** im vollständig expandierten Zustand mindestens 50 mm beträgt, wobei das selbstexpandierbare geflochtene Gerüst **(20)** eine Vielzahl von Schichten **(22, 23, 24)** aus Drähten **(25)** aus einem biokompatiblen Material umfasst, jede Schicht ein Maschengeflecht bildet, die Maschengeflechte ein Gitter mit einer Vielzahl von Drähten **(25)** gegebener Schichten **(22, 23, 24)** bilden, das Gitter, wenn es normal zu einer Wand des selbstexpandierbaren geflochtenen Gerüsts **(20)** betrachtet wird, polygonale Öffnungseinheiten **(26)** definiert, und ein Verhältnis ($T_{20}/\varnothing_{25}$) der Dicke ($T_{20}$) einer Wand des selbstexpandierbaren geflochtenen Gerüsts **(20)** zum Durchmesser ($\varnothing_{25}$) des Drahts **(25)** höher als 2,0 ist.

2.  Implantierbare endoluminale Prothese **(1)** nach Anspruch 1, wobei die Maschengeflechte miteinander verriegelt sind und ein Gitter mit einer Vielzahl von Drähten gegebener Schichten bilden, wobei die Drähte im Maschengeflecht mindestens einer der benachbarten Schichten integriert sind, so dass die Maschengeflechte benachbarter Schichten des Gerüsts im Wesentlichen versetzt sind.

3.  Implantierbare endoluminale Prothese **(1)** nach Anspruch 1 oder 2, wobei die Gesamtlänge des röhrenförmigen Hauptkörpers **(3)** und des Übergangsabschnitts **(4)** im vollständig expandierten Zustand mindestens 100 mm, vorzugsweise 150 mm und insbesondere 200 mm beträgt.

4.  Implantierbare endoluminale Prothese **(1)** nach einem der vorhergehenden Ansprüche, wobei das Verhältnis ($T_{20}/\varnothing_{25}$) mindestens 3,5, insbesondere 5,5, besonders bevorzugt 6,5, noch mehr bevorzugt 7,5 beträgt.

5.  Implantierbare endoluminale Prothese **(1)** nach einem der vorhergehenden Ansprüche, wobei das selbstexpandierbare geflochtene Gerüst **(20)** aus mindestens 150 Drähten, insbesondere mindestens 180 Drähten, besonders bevorzugt mindestens 250 Drähten, noch mehr bevorzugt mindestens 300 Drähten besteht.

6.  Implantierbare endoluminale Prothese **(1)** nach einem der vorhergehenden Ansprüche, wobei der Durchmesser des Drahts **(25)** mindestens 30 μm und höchstens 180 μm, vorzugsweise mindestens 50 μm und höchstens 150 μm, insbesondere mindestens 75 μm und höchstens 100 μm beträgt.

7.  Implantierbare endoluminale Prothese **(1)** nach einem der vorhergehenden Ansprüche, wobei ein Flächenabdeckungsverhältnis (SCR) des selbstexpandierbaren geflochtenen Gerüsts **(20)** in einem vollständig expandierten Zustand mindestens 35%, vorzugsweise mindestens 45%, insbesondere mindestens 55%, besonders bevorzugt mindestens 65% und weniger als 90% beträgt.

8.  Implantierbare endoluminale Prothese **(1)** nach einem der vorhergehenden Ansprüche, wobei ein mittlerer Durchmesser ($\varnothing_{27}$) eines Innenkreises **(27)** der polygonalen Öffnungseinheiten **(26)** im vollständig expandierten Zustand mindestens 50 μm und höchstens 200 μm, vorzugsweise mindestens 100 μm und höchstens 150 μm beträgt.

9.  Implantierbare endoluminale Prothese **(1)** nach einem der vorhergehenden Ansprüche, wobei, wenn die implantierbare endoluminale Prothese **(1)** in einem gebogenen Lumen mit einem H/B-Verhältnis zwischen 0,5 und 0, 9 eingesetzt wird, der mittlere Durchmesser ($\varnothing_{27}$) des Innenkreises **(27)** der polygonalen Öffnungseinheiten **(26)** mindestens 50 μm und höchstens 250 μm beträgt, wobei ein längenbezogenes Kompressionsverhältnis (LCR) zwischen 15% und 40% beträgt und das Flächenabdeckungsverhältnis (SCR) des selbstexpandierbaren geflochtenen Gerüsts **(20)** mehr als 35% auf der Seite der Außenkurve beträgt.

10. Implantierbare endoluminale Prothese **(1)** nach einem der vorhergehenden Ansprüche, wobei der Übergangsabschnitt **(4)** einen Querschnitt mit einem Durchmesser aufweist, der größer als der des röhrenförmigen Hauptkörpers **(3)** ist, um eine kugelförmige Gestalt zu bilden.

11. Implantierbare endoluminale Prothese **(1)** nach einem der vorhergehenden Ansprüche, wobei das selbstexpandierbare geflochtene Gerüst **(20)** ferner einen Dichtungsabschnitt **(8)** zwischen dem proximalen Ende **(6)** des geflochtenen Gerüsts und dem Hals **(5)** umfasst, wobei der Durchmesser des Dichtungsabschnitts **(8)** zum proximalen Ende **(6)** des geflochtenen Gerüsts hin zunimmt.

12. Implantierbare endoluminale Prothese **(1)** nach einem der vorhergehenden Ansprüche, wobei das selbstexpandierbare geflochtene Gerüst **(20)** ferner einen vergrößerten Abschnitt **(2)** zwischen dem distalen Ende **(7)** des selbstexpandierbaren geflochtenen Gerüsts **(20)** und dem röhrenförmigen Hauptkörper **(3)** umfasst, wobei der Durchmesser des vergrößerten Abschnitts zum distalen Ende **(7)** des selbstexpandierbaren geflochtenen Gerüsts **(20)** hin zunimmt.

13. Implantierbare endoluminale Prothese nach einem der vorhergehenden Ansprüche, wobei das biokompatible Material ein Metallsubstrat ist, das aus der aus Titan, Nickel-Titan-Legierungen, wie z.B. Nitinol und Nitinol-DFT®-Platin, eine beliebige Art von Edelstahlen oder Kobalt-Chrom-Nickel-Legierungen, wie z.B. Phynox® bestehenden Gruppe ausgewählt ist.

14. Implantierbare endoluminale Prothese **(1)** nach Anspruch 13, wobei die Oberfläche der Drähte mit einem Gem-Bisphosphonat abgedeckt ist, so dass mindestens ein Phosphonat-Rest kovalent und direkt mit der Außenseite des Drahts **(25)** verbunden ist, und wobei das Gem-Bisphosphonat mindestens 50% der Außenseite der Drähte **(25)** als Monoschicht und als äußerste Schicht bedeckt.

15. Implantierbare endoluminale Prothese **(1)** nach Anspruch 14, wobei die Oberfläche der Drähte mit Phosphonat beschichtet ist, das eine Kohlenwasserstoffkette mit 3 bis 16 Kohlenstoffatomen als lineare Kette umfasst, wobei das Phosphoratom des Phosphonats an der alpha-Position an die Kohlenwasserstoffkette gebunden ist, wobei die Kohlenwasserstoffkette ferner an ihrer terminalen Position von einer Carboxylgruppe, einer Phosphongruppe oder einer Hydroxylgruppe funktionalisiert ist, wobei das Phosphonat kovalent und direkt mit der Außenseite des Drahts **(25)** verbunden ist und mindestens 50% der Außenseite der Drähte **(25)** als Monoschicht und als äußerste Schicht bedeckt.

16. Implantierbare endoluminale Prothese **(1)** nach einem der vorhergehenden Ansprüche zur Verwendung bei der Verhütung eines embolischen Schlaganfalls für Patienten während und nach der Implantation einer Prothesenklappe, durch Abdeckung von Öffnungen der Koronararterien und der supraaortalen Äste, die Blut zum Herz und Gehirn leiten, mit der implantierbaren endoluminalen Prothese (**1**).

17. Implantierbare endoluminale Prothese **(1)** nach einem der Ansprüche 1 bis 9 zur Verwendung bei der Verbesserung der Durchblutung eines Organs durch Abdeckung von Öffnungen der Koronararterien und der supraaortalen Äste, die Blut zum Herz und Gehirn leiten, mit der implantierbaren endoluminalen Prothese (**1**).

## Revendications

1. Prothèse endoluminale implantable (1) appropriée pour déploiement à partir de l'anneau aortique jusqu'à l'aorte, comprenant:

   1) un canevas tressé auto-expansible (20) capable de s'expanser à partir d'un état radialement comprimé dans une configuration de pose jusqu'à un état radialement expansé, le canevas tressé auto-expansible (20) étant constitué de fils tressés présentant un diamètre donné ($\varnothing_{25}$) et présentant une extrémité proximale (6) configurée de manière à s'étendre en direction du coeur et une extrémité distale (7) configurée de manière à s'étendre à l'écart du coeur et s'étendant le long d'un axe, le canevas tressé auto-expansible (20) comprenant:

   a) à l'extrémité distale (7) du canevas tressé auto-expansible (20), un corps tubulaire principal (3) comportant une lumière de forme cylindrique présentant une section transversale circulaire et un diamètre constant;
   b) à l'extrémité proximale (6) du canevas tressé auto-expansible (20), un goulot (5) comportant une lumière de forme cylindrique présentant une section transversale circulaire et un diamètre constant plus petit que celui dudit corps tubulaire principal (3); et
   c) une partie de transition (4) s'étendant entre l'extrémité proximale (6) du corps tubulaire principal (3) et l'extrémité distale du goulot (5), ledit corps tubulaire principal (3), ledit goulot (5) et ladite partie de transition (4) étant constitués d'une structure intégrée dépourvue de toute couche de recouvrement imperméable, et formant une paroi présentant une épaisseur ($T_{20}$),

   2) un corps de valve radialement compressible (10) comprenant un matériau imperméable placé à l'intérieur de la lumière du goulot (5),

   **caractérisée en ce que**, dans l'état complètement expansé, la longueur totale du corps tubulaire principal (3) et de la partie de transition (4) est d'au moins 50 mm, le canevas tressé auto-expansible (20) comprenant une pluralité de couches (22, 23, 24) de fils (25) constitués d'un matériau biocompatible, chaque couche formant un maillage, les maillages formant un treillis comprenant une pluralité de fils (25) de couches données (22, 23, 24), le treillis, lorsqu'il est observé normalement à une paroi du canevas tressé auto-expansible (20), définissant des unités d'ouverture polygonales (26), un rapport ($T_{20}/\varnothing_{25}$) entre

l'épaisseur ($T_{20}$) d'un paroi du canevas tressé auto-expansible (20) et le diamètre ($\varnothing_{25}$) de fil (25) étant supérieur à 2,0.

2. Prothèse endoluminale implantable (1) selon la revendication 1, dans laquelle les maillages sont entremêlés pour former un treillis comprenant une pluralité de fils de couches données, les fils étant intégrés dans le maillage d'au moins une des couches adjacentes de telle sorte que des maillages de couches adjacentes du canevas soient sensiblement décalés.

3. Prothèse endoluminale implantable (1) selon l'une ou l'autre des revendications 1 ou 2, dans laquelle, dans l'état complètement expansé, la longueur totale du corps tubulaire principal (3) et de la partie de transition (4) est d'au moins 100 mm, de préférence de 150 mm, mieux encore de 200 mm.

4. Prothèse endoluminale implantable (1) selon l'une quelconque des revendications précédentes, dans laquelle le rapport ($T_{20}/\varnothing_{25}$) est d'au moins 3,5, mieux encore de 5,5, mieux encore même de 6,5, encore mieux encore même de 7,5.

5. Prothèse endoluminale implantable (1) selon l'une quelconque des revendications précédentes dans laquelle le canevas tressé auto-expansible (20) est constitué d'au moins 150 fils, mieux encore d'au moins 180 fils, mieux encore même d'au moins 250 fils, encore mieux encore même d'au moins 300 fils.

6. Prothèse endoluminale implantable (1) selon l'une quelconque des revendications précédentes, dans laquelle le diamètre de fil (25) est d'au moins 30 $\mu$m et d'au plus 180 $\mu$m, de préférence d'au moins 50 $\mu$m et d'au plus 150 $\mu$m, mieux encore d'au moins 75 $\mu$m et d'au plus 100 $\mu$m.

7. Prothèse endoluminale implantable (1) selon l'une quelconque des revendications précédentes, dans laquelle, dans un état complètement expansé, un rapport de couverture de surface (SCR) dudit canevas tressé auto-expansible (20) est d'au moins 35 %, de préférence d'au moins 45 %, mieux encore d'au moins 55 %, encore mieux encore d'au moins 65 % et inférieur à 90 %.

8. Prothèse endoluminale implantable (1) selon l'une quelconque des revendications précédentes, dans laquelle un diamètre moyen ($\varnothing_{27}$) d'un cercle inscrit (27) des unités d'ouverture polygonales (26) est, dans l'état complètement expansé, d'au moins 50 $\mu$m et d'au plus 200 $\mu$m, de préférence d'au moins 100 $\mu$m et d'au plus 150 $\mu$m.

9. Prothèse endoluminale implantable (1) selon l'une quelconque des revendications précédentes, dans laquelle, lorsque la prothèse endoluminale implantable (1) est déployée dans une lumière courbe présentant un rapport hauteur/largeur compris entre 0,5 et 0,9, le diamètre moyen ($\varnothing_{27}$) du cercle inscrit (27) des unités d'ouverture polygonales (26) est d'au moins 50 $\mu$m et d'au plus 250 $\mu$m, un rapport de compression relatif à la longueur (LCR) est compris entre 15 % et 40 %, et le rapport de couverture de surface (SCR) du canevas tressé auto-expansible (20) est supérieur à 35 % sur le côté de la courbe extérieure.

10. Prothèse endoluminale implantable (1) selon l'une quelconque des revendications précédentes, dans laquelle la partie de transition (4) présente une section transversale dont le diamètre est plus grand que celui du corps tubulaire principal (3) de manière à à former une forme globulaire.

11. Prothèse endoluminale implantable (1) selon l'une quelconque des revendications précédentes, dans laquelle le canevas tressé auto-expansible (20) comprend en outre une partie d'étanchéité (8) entre l'extrémité proximale (6) du canevas tressé et le goulot (5), le diamètre de la partie d'étanchéité (8) augmentant en direction de l'extrémité proximale (6) du canevas tressé.

12. Prothèse endoluminale implantable (1) selon l'une quelconque des revendications précédentes, dans laquelle le canevas tressé auto-expansible (20) comprend en outre une partie agrandie (2) entre l'extrémité distale (7) du canevas tressé auto-expansible (20) et le corps tubulaire principal (3), le diamètre de la partie agrandie augmentant en direction de l'extrémité distale (7) du canevas tressé auto-expansible (20).

13. Prothèse endoluminale implantable selon l'une quelconque des revendications précédentes, dans laquelle le matériau biocompatible est un substrat métallique sélectionné dans le groupe comprenant le titane, des alliages de nickel et de titane tels que le nitinol et le Nitinol-DFT®-Platinum, tous les types d'acier inoxydable, ou des alliages de cobalt, de chrome et de nickel tels que le Phynox®.

14. Prothèse endoluminale implantable (1) selon la revendication 13, dans laquelle la surface desdits fils est recouverte avec un gem-bisphosphonate de telle sorte qu'au moins un fragment de phosphonate soit lié de façon covalente et directe à la surface externe du fil (25), et le gem-bisphosphonate couvrant au moins 50 % de la surface externe des fils (25) comme une monocouche et faisant office de couche extérieure ultime.

**15.** Prothèse endoluminale implantable (1) selon la revendication 14, dans laquelle la surface desdits fils est revêtue de phosphonate contenant une chaîne hydrocarbonée comprenant de 3 à 16 atomes de carbone comme chaîne linéaire, l'atome de phosphore du phosphonate se liant à la chaîne hydrocarbonée à la position alpha, ladite chaîne hydrocarbonée étant en outre fonctionnalisée à sa position terminale par un groupe carboxylique, un groupe phosphonique ou un groupe hydroxyle, le phosphonate étant lié de façon covalente et directe à la surface externe du fil (25) et couvrant au moins 50 % de la surface externe des fils (25) comme une monocouche et faisant office de couche extérieure ultime.

**16.** Prothèse endoluminale implantable (1) selon l'une quelconque des revendications précédentes à utiliser pour la prévention de l'embolie cérébrale chez des patients pendant et après l'implantation de valves prothétiques, en couvrant avec ladite prothèse endoluminale implantable (1) des orifices des coronaires et des branches supra-aortiques qui transportent le sang jusqu'au coeur et au cerveau.

**17.** Prothèse endoluminale implantable (1) selon l'une quelconque des revendications 1 à 9 à utiliser pour améliorer la perfusion d'un organe en couvrant avec ladite prothèse endoluminale implantable (1) des orifices des coronaires et des branches supra-aortiques qui transportent le sang jusqu'au coeur et au cerveau.

Fig. 1

Fig. 2a

Fig. 2b

Fig. 2c

EP 3 078 350 B1

Fig. 3

Fig. 4a

Fig. 4b

Fig. 5

Fig. 6

EP 3 078 350 B1

Fig. 7

EP 3 078 350 B1

Fig. 8

Fig. 9

Fig. 10

Fig. 11

EP 3 078 350 B1

Fig. 13a

Fig. 13

Fig. 12a

Fig. 12

Fig. 14

37

31

39

H30

W30

Fig. 15

Fig. 16a

Fig. 16

Fig. 17a

22

35

27

Fig. 17b

23

22

35

27

Fig. 17c

22

23

24

35

27

Fig. 18a

22

35

Fig. 18b

23

22

35

Fig. 18c

35

23

23

24

EP 3 078 350 B1

Fig. 19b (Prior Art)

Fig. 19a (Prior Art)

Fig. 20

Fig. 21a

Fig. 21b

EP 3 078 350 B1

## Fig. 22

MEAN $\emptyset_{27}$
OF POLYGON
OPENING
(µm)

H/W RATIO

Fig. 23

EP 3 078 350 B1

## Fig. 24

MEAN $\varnothing_{27}$
OF POLYGON
OPENING
(µm)

150

50

0,5        0,9        H/W RATIO

## Fig. 25

MEAN Ø₂₇
OF POLYGON
OPENING
(µm)

150

50

COMPRESSION
RATIO
40 (%)

30

0,5

0,9

H/W RATIO

EP 3 078 350 B1

## REFERENCES CITED IN THE DESCRIPTION

**Patent documents cited in the description**

- US 6673089 B
- US 6740112 B
- US 20030100940 A
- US 5061275 A
- US 2013144373 A
- EP 1248372 A
- US 20060015138 A

**Non-patent literature cited in the description**

- **OAKE N ; FERGUSSON DA ; FORSTER AJ ; VAN WALRAVEN C.** Frequency of adverse events in patients with poor anticoagulation: a meta-analysis. *CMAJ,* 2007, vol. 176 (11), 1589-94
- **SIEVERT et al.** *Cardiovas Intervent Radiol,* 2012, vol. 35, 406-412
- **OU et al.** *J. Thrac. Cardiovasc. Surg.,* 2006, vol. 132, 1105-1111
- Reduction of cerebral embolixation in carotid angioplasty: An in-vitro experiment comparing 2 cerebral protection devices. J. Endovasc. Ther. Charalambous, 2009, vol. 16, 161-167